(19) Europäisches Patentamt
European Patent Office
Office européen des brevets

(11) EP 2 589 591 A1

(12) EUROPEAN PATENT APPLICATION
published in accordance with Art. 153(4) EPC

(43) Date of publication:
**08.05.2013 Bulletin 2013/19**

(21) Application number: **11800456.3**

(22) Date of filing: **30.06.2011**

(51) Int Cl.:
***C07D 209/86*** *(2006.01)* ***C07D 401/14*** *(2006.01)*
***C07D 403/14*** *(2006.01)* ***C07D 409/14*** *(2006.01)*
***H01L 51/50*** *(2006.01)*

(86) International application number:
**PCT/JP2011/003764**

(87) International publication number:
**WO 2012/001986 (05.01.2012 Gazette 2012/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2010 JP 2010148695**

(71) Applicant: **Hodogaya Chemical Co., Ltd.**
**Tokyo 105-0011 (JP)**

(72) Inventors:
- **YOKOYAMA, Norimasa**
  **Chuo-ku**
  **Tokyo 1040028 (JP)**
- **NAGAOKA, Makoto**
  **Tsukuba-shi**
  **Ibaraki 305-0841 (JP)**
- **KABASAWA, Naoaki**
  **Chuo-ku**
  **Tokyo 1040028 (JP)**
- **IZUMI, Sawa**
  **Chuo-ku**
  **Tokyo 1040028 (JP)**
- **TAKAHASHI, Eiji**
  **Tsukuba-shi**
  **Ibaraki 305-0841 (JP)**

(74) Representative: **Müller-Boré & Partner**
**Patentanwälte**
**Grafinger Straße 2**
**81671 München (DE)**

(54) **COMPOUND WITH CARBAZOLE RING STRUCTURE AND ORGANIC ELECTROLUMINESCENT ELEMENT**

(57) There is provided an organic compound of excellent characteristics that exhibits excellent hole-injecting/transporting performance and has high triplet exciton confining capability with an electron blocking ability, and that has high stability in the thin-film state and high luminous efficiency. The compound is used to provide a high-efficiency, high-durability organic electroluminescent device, particularly a phosphorescent organic electroluminescent device.

The present invention is a compound of the following general formula having a carbazole ring structure. The compound is used as a constituent material of at least one organic layer in an organic electroluminescent device that includes a pair of electrodes, and one or more organic layers sandwiched between the pair of electrodes.

[Chemical Formula 1]

EP 2 589 591 A1

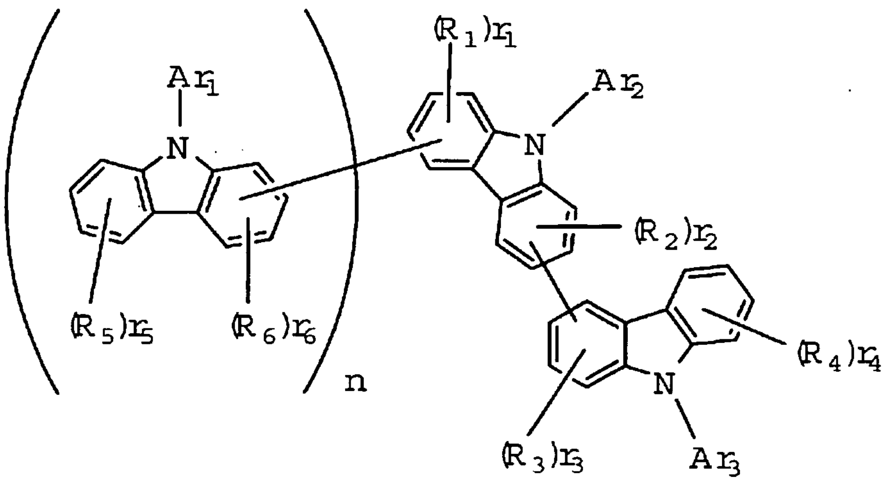

## Description

TECHNICAL FIELD

The present invention relates to compounds suited for an organic electroluminescent device (hereinafter, simply referred to as "organic EL device"), a preferred self light-emitting device for various display devices, and to the device. Specifically, the invention relates to compounds having a carbazole ring structure, and to organic EL devices that use the compounds.

Background Art

The organic EL device is a self-emitting device, and has been actively studied for their brighter, superior viewability and ability to display clearer images compared with the liquid crystal device.

In 1987, C. W. Tang et al. at Eastman Kodak developed a laminated structure device using materials assigned with different roles, realizing practical applications of an organic EL device with organic materials. These researchers laminated tris(8-hydroxyquinoline)aluminum (an electron-transporting phosphor; hereinafter, simply $Alq_3$), and a hole-transporting aromatic amine compound, and injected the both charges into the phosphor layer to cause emission in order to obtain a high luminance of 1,000 cd/$m^2$ or more at a voltage of 10 V or less (see, for example, Patent Documents 1 and 2).

To date, various improvements have been made for practical applications of the organic EL device. In order to realize high efficiency and durability, various roles are further subdivided to provide an electroluminescent device that includes an anode, a hole injection layer, a hole transport layer, a light emitting layer, an electron transport layer, an electron injection layer, and a cathode successively formed on a substrate (see, for example, Non-Patent Document 1).

Further, there have been attempts to use triplet excitons for further improvements of luminous efficiency, and use of phosphorescent materials has been investigated (see, for example, Non-Patent Document 2).

The light emitting layer can also be fabricated by doping a charge-transporting compound, generally called a host material, with a phosphor or a phosphorescent material. As described in the foregoing lecture preprints, selection of organic materials in an organic EL device greatly influences various device characteristics, including efficiency and durability.

In an organic EL device, the charges injected from the both electrodes recombine at the light emitting layer to cause emission. Here, it is important how efficiently the hole and electron charges are transferred to the light emitting layer. The probability of hole-electron recombination can be improved by improving the hole injectability and the electron blocking performance of blocking the injected electrons from the cathode, and high luminous efficiency can be obtained by confining the excitons generated in the light emitting layer. The role of the hole transport material is therefore important, and there is a need for a hole transport material that has high hole injectability, high hole mobility, high electron blocking performance, and high electron durability.

There is also a need for a hole transport material that is stable as a thin film, and has high heat resistance.

The aromatic amine derivatives described in Patent Documents 1 and 2 are known examples of the hole transport materials used for the organic EL device. These compounds include a compound known to have an excellent hole mobility of $10^{-3}$ $cm^2/Vs$ or higher. However, the compound is insufficient in terms of electron blocking performance, and some of the electrons pass through the light emitting layer. Accordingly, improvements in luminous efficiency cannot be expected.

Arylamine compounds of the following formulae having a substituted carbazole structure (for example, Compounds A, B, and C) are proposed as improvements over the foregoing compounds (see, for example, Patent Documents 3 to 5).

[Chemical Formula 1]

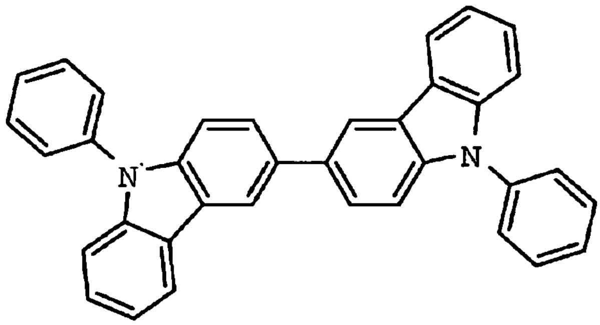

(Compound A)

[Chemical Formula 2]

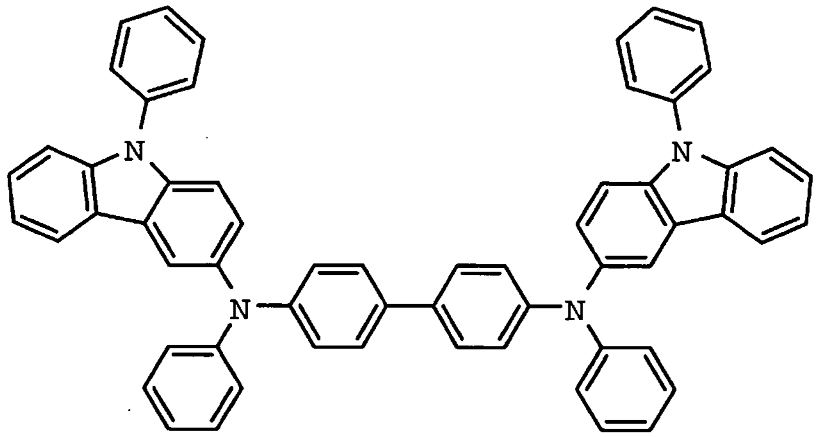

(Compound B)

[Chemical Formula 3]

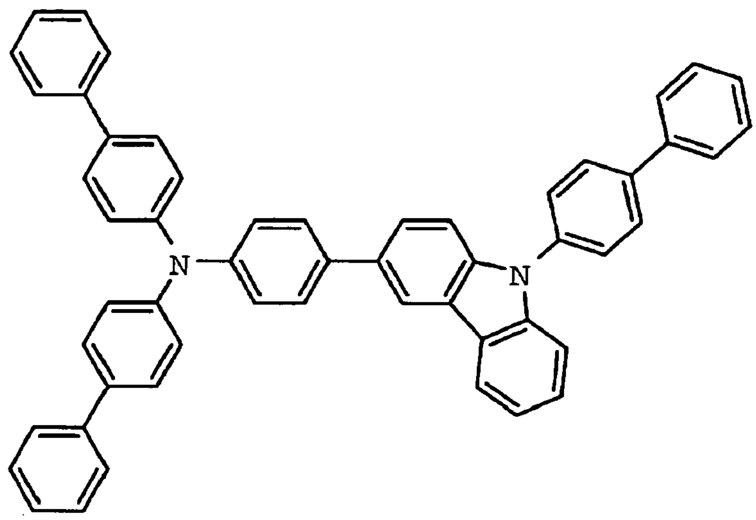

(Compound C)

In an attempt to improve the device luminous efficiency, there have been developed devices that use phosphorescent materials to generate phosphorescence, specifically that make use of the emission from the triplet excitation state. According to the excitation state theory, phosphorescent materials are expected to greatly increase the luminous efficiency about four times as much as that of the conventional fluorescence.
In 1999, M.A. Baldo et al. at Princeton University realized 8% luminous efficiency with a phosphorescent device using an iridium complex, a great improvement over the conventional external quantum efficiency. The phosphorescent device has been actively developed ever since.
Improving the luminous efficiency of the phosphorescent device requires use of materials of high excitation triplet energy level (hereinafter, simply "$T_1$") for the host material. However, there is a report that use of materials with high $T_1$ is also necessary for the hole transport material to confine the triplet excitons (see, for example, Non-Patent Document 3). Further, the green phosphorescent material tris(phenylpyridyl)iridium (hereinafter, simply "$Ir(ppy)_3$") represented by the following formula has a $T_1$ of 2.42 eV.
[Chemical Formula 4]

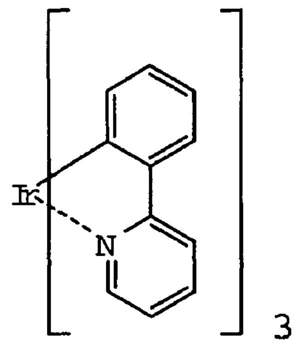

Because N,N'-diphenyl-N,N'-di(α-naphthyl)benzidine (hereinafter, simply "α-NPD") has a $T_1$ of 2.29 eV, sufficient confinement of the triplet excitons cannot be expected with α-NPD. Higher luminous efficiency is thus obtained using 1,1-bis[4-(di-4-tolylamino)phenyl]cyclohexane (hereinafter, simply "TAPC") of the following formula having a higher $T_1$ value of 2.9 eV (see, for example, Non-Patent Document 4).
[Chemical Formula 5]

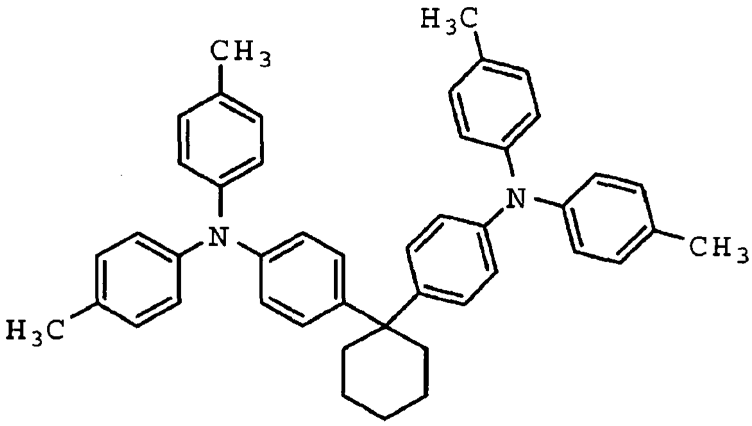

However, the TAPC has low hole mobility, and its ionization potential (work function) 5.8 eV is not appropriate for a hole transport material.

The ionization potential (work function) of Compound A is 5.5 eV, a more appropriate value compared to the ionization potential of the TAPC. It is expected that this, combined with the high $T_1$ of 2.9 eV, would provide sufficient confinement of the triplet excitons. However, because the compound has low hole mobility, the product device has high driving voltage, and the luminous efficiency cannot be said as sufficient (see, for example, Non-Patent Document 5). Accordingly, there is a need for materials having a high $T_1$ value and high hole mobility that can be used as a hole injection layer or a hole transport layer, in order to obtain a phosphorescent device having improved luminous efficiency.

There is a report of a high-efficient organic EL device obtained by using a deuterium atom-substituted carbazole compound as the material of the light emitting layer (see, for example, Patent Documents 6 and 7). This is an application of the principle that the luminous efficiency increases by facilitating the formation of excitons when substituted with deuterium atom. While this is true for the material of the light emitting layer, the technique cannot be applied to the material of the hole transport layer, the hole injection layer, or the electron blocking layer that does not directly involve in the exciton formation. In fact, there is no known example of an application to the material of the hole transport layer.

Citation List

Patent Documents

Patent Document 1: JP-A-8-048656
Patent Document 2: Japanese Patent Number 3194657
Patent Document 3: JP-A-8-003547
Patent Document 4: JP-A-2006-151979
Patent Document 5: WO2008/62636
Patent Document 6: JP-A-2005-048004
Patent Document 7: JP-A-2009-231516
Patent Document 8: JP-A-2007-022986


Non-Patent Documents

Non-Patent Document 1: The Japan Society of Applied Physics, 9th lecture preprints, pp. 55 to 61 (2001)
Non-Patent Document 2: The Japan Society of Applied Physics, 9th lecture preprints, pp. 23 to 31 (2001)
Non-Patent Document 3: J. Appl. Phys., 12, 95, 7798 (2004)
Non-Patent Document 4: Organic EL Display, 89 (2004), Tokitoh, Adachi, Murata, Ohmsha
Non-Patent Document 5: Appl. Phys. Lett., 93, 063306 (2008)
Non-Patent Document 6: Helvetica Chimica Acta., vol.89, 1123 (2006)
Non-Patent Document 7: J. Org. Chem., 60, 7508 (1995)

Non-Patent Document 8: Synth. Commun., 11, 513 (1981)
Non-Patent Document 9: Jikken Kagaku Kouza 7, 4th ed., pp. 384-398 (1992), The Chemical Society of Japan, Maruzen
Non-Patent Document 10: Organic EL Symposium, the 1st Regular presentation Preprints, 19 (2005)
Non-Patent Document 11: Appl. Phys. Lett., 93, 133312 (2008)

Summary of the Invention

Problems that the Invention is to Solve

It is an object of the present invention to provide an organic compound of excellent characteristics that exhibits excellent hole-injecting/transporting performance and has high triplet exciton confining capability with an electron blocking ability, and that has high stability in the thin-film state and high luminous efficiency. The invention also provides a high-efficient, high-durable organic EL device, particularly a phosphorescent organic EL device, using the compound.
Some of the physical properties of the organic compound used for the organic EL device of the present invention include (1) good hole injection characteristics, (2) high hole mobility, (3) high $T_1$ value, (4) excellent electron blocking ability, (5) stability in the thin-film state, and (6) excellent heat resistance. Some of the physical properties of the organic EL device to be provided by the present invention include (1) high luminous efficiency and high power efficiency, (2) low turn on voltage, and (3) low actual driving voltage.

Means for Solving the Problems

In order to achieve the foregoing objects, the present inventors focused on the high $T_1$ value, the excellent electron blocking performance and excellent hole transporting ability of a carbazole ring structure, and produced various test organic EL devices by designing, selecting, and chemically synthesizing compounds linked to a carbazole ring structure, in anticipation that the carbazole ring structure, upon substitution with a deuterium atom, would effectively improve heat resistance and thin film stability. The present invention was completed after thorough evaluations of the device characteristics.
1) Specifically, the present invention is a compound of the following general formula (1) having a carbazole ring structure.
[Chemical Formula 6]

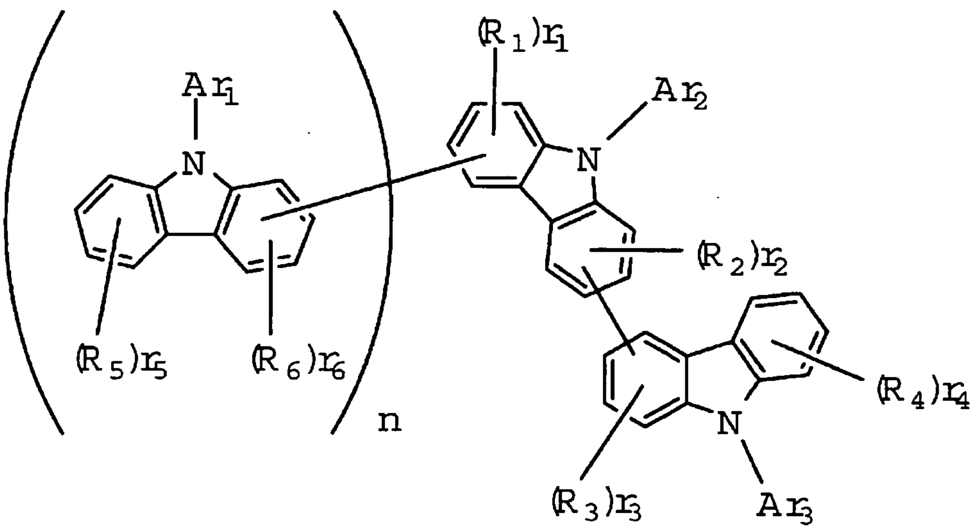

(1)

In the formula, $R_1$, $R_2$, $R_3$, $R_4$, $R_5$, and $R_6$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to

6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. $r_1$, $r_4$, and $r_5$ may be the same or different, and represent 0 or an integer of 1 to 4. $r_2$, $r_3$, and $r_6$ may be the same or different, and represent 0 or an integer of 1 to 3. n represents 0 or an integer of 1. $Ar_1$, $Ar_2$, and $Ar_3$ may be the same or different, and represent a substituted or unsubstituted aromatic hydrocarbon group, a substituted or unsubstituted aromatic heterocyclic group, or a substituted or unsubstituted condensed polycyclic aromatic group. At least one of $R_1$ to $R_6$, or at least one of the substituents of $Ar_1$ to $Ar_3$ is a deuterium atom, or a substituent that contains a deuterium atom.

2) Further, the present invention is a compound of the general formula (1) having a carbazole ring structure in which $Ar_2$ is a monovalent group represented by the following general formula (2) or (3).

[Chemical Formula 7]

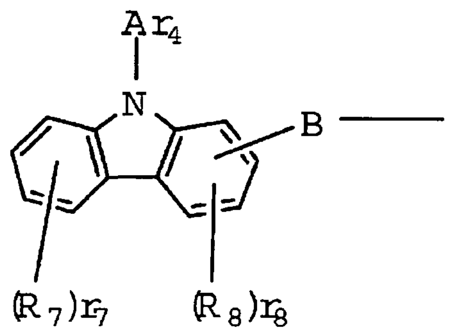

(2)

In the formula, $R_7$ and $R_8$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. $r_7$ represents 0 or an integer of 1 to 4, and $r_8$ represents 0 or an integer of 1 to 3. B represents a divalent group of a substituted or unsubstituted aromatic hydrocarbon group, a divalent group of a substituted or unsubstituted aromatic heterocyclic ring, or a divalent group of a substituted or unsubstituted condensed polycyclic aromatic group. $Ar_4$ represents a substituted or unsubstituted aromatic hydrocarbon group, a substituted or unsubstituted aromatic heterocyclic group, or a substituted or unsubstituted condensed polycyclic aromatic group. At least one of $R_7$ and $R_8$, or at least one of the substituents of $Ar_4$ or B is a deuterium atom, or a substituent that contains a deuterium atom.

[Chemical Formula 8]

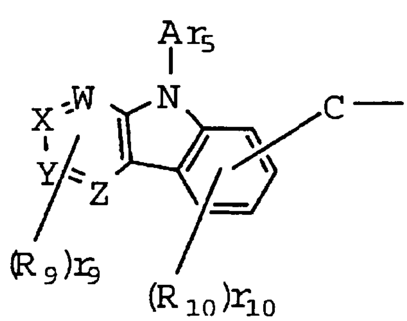

(3)

In the formula, $R_9$ and $R_{10}$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. $r_9$ and $r_{10}$ may be the same or different, and represent 0 or an integer of 1 to 3. C represents a divalent group of a substituted or unsubstituted aromatic hydrocarbon group, a divalent group of a substituted or unsubstituted aromatic heterocyclic ring, or a divalent group of a substituted or unsubstituted condensed polycyclic aromatic group. $Ar_5$ represents a substituted or unsubstituted aromatic hydrocarbon group, a substituted or unsubstituted aromatic heterocyclic group, or a substituted or unsubstituted condensed polycyclic aromatic group. W, X, Y, and Z represent a carbon atom or a nitrogen atom, where only one of W, X, Y, and Z is a nitrogen atom, and, in this case, the nitrogen atom does not have the substituent $R_9$. At least one of $R_9$ and $R_{10}$, or at least one of the substituents of $Ar_5$ or C is a deuterium atom, or a substituent that contains a deuterium atom.

3) Further, the present invention is a compound of the general formula (1) having a carbazole ring structure according to 1) or 2), wherein n in the general formula (1) is 0.

4) Further, the present invention is a compound of the general formula (1) having a carbazole ring structure according to 1) or 2), wherein n in the general formula (1) is 1.

5) Further, the present invention is an organic EL device that includes a pair of electrodes, and one or more organic layers sandwiched between the pair of electrodes, wherein the compound having a carbazole ring structure according to 1) to 4) is used as a constituent material of at least one organic layer.

6) Further, the present invention is an organic EL device according to 5) in which the organic layer is a hole transport layer.

7) Further, the present invention is an organic EL device according to 5) in which the organic layer is a hole injection layer.

8) Further, the present invention is an organic EL device according to 5) in which the organic layer is an electron blocking layer.

9) Further, the present invention is an organic EL device according to any one of 5) to 8) that further includes a light emitting layer containing a phosphorescent light-emitting material.

10) Further, the present invention is an organic EL device according to 9) in which the phosphorescent light-emitting material is a metal complex that contains iridium or platinum.

Specific examples of the "linear or branched alkyl of 1 to 6 carbon atoms" or the "cycloalkyl of 5 to 10 carbon atoms" in the "linear or branched alkyl of 1 to 6 carbon atoms that may have a substituent" or the "cycloalkyl of 5 to 10 carbon atoms that may have a substituent" represented by $R_1$ to $R_{10}$ in the general formulae (1) to (3) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, and 2-adamantyl.

Specific examples of the "substituent" in the "linear or branched alkyl of 1 to 6 carbon atoms having a substituent" or the "cycloalkyl of 5 to 10 carbon atoms having a substituent" represented by $R_1$ to in the general formulae (1) to (3) include a deuterium atom, a fluorine atom, a chlorine atom, cyano, trifluoromethyl, nitro, phenyl, naphthyl, anthryl, styryl, phenoxy, tolyloxy, benzyloxy, and phenethyloxy. These substituents may be further substituted.

Specific examples of the "linear or branched alkyloxy of 1 to 6 carbon atoms" or the "cycloalkyloxy of 5 to 10 carbon atoms" in the linear or branched alkyloxy of 1 to 6 carbon atoms that may have a substituent" or the "cycloalkyloxy of 5 to 10 carbon atoms that may have a substituent" represented by $R_1$ to $R_{10}$ in the general formulae (1) to (3) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy.

Specific examples of the "substituent" in the "linear or branched alkyloxy of 1 to 6 carbon atoms having a substituent" or the "cycloalkyloxy of 5 to 10 carbon atoms having a substituent" represented by $R_1$ to $R_{10}$ in the general formulae (1) to (3) include a deuterium atom, a fluorine atom, a chlorine atom, cyano, trifluoromethyl, nitro, phenyl, naphthyl, anthryl, styryl, phenoxy, tolyloxy, benzyloxy, and phenethyloxy. These substituents may be further substituted.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by $R_1$ to $R_{10}$ or $Ar_1$ to $Ar_5$ in general formulae (1) to (3) include phenyl, biphenylyl, terphenylyl, naphthyl, anthryl, phenanthryl, fluorenyl, indenyl, pyrenyl, acenaphthenyl, fluoranthenyl, triphenylenyl, pyridyl, furanyl, pyranyl, thienyl, quinolyl, isoquinolyl, benzofuranyl, benzothienyl, indolyl, carbazolyl, benzooxazolyl, benzothiazolyl, quinoxalyl, benzoimidazolyl, pyrazolyl, dibenzofuranyl, dibenzothienyl, and carbolinyl, of which phenyl, biphenylyl, terphenylyl, fluorenyl, carbazolyl, and carbolinyl are preferable. Preferably, the "condensed polycyclic aromatic group" has 20 or less carbon atoms, because $T_1$ becomes smaller as the number of carbon atoms in the "condensed polycyclic aromatic group" increases.

Specific examples of the "substituent" in the "substituted aromatic hydrocarbon group", the "substituted aromatic heterocyclic group", or the "substituted condensed polycyclic aromatic group" represented by $R_1$ to $R_{10}$ or $Ar_1$ to $Ar_5$ in general formulae (1) to (3) include a deuterium atom, a fluorine atom, a chlorine atom, cyano, trifluoromethyl, nitro, linear or branched alkyl of 1 to 6 carbon atoms, cycloalkyl of 5 to 10 carbon atoms, linear or branched alkenyl of 2 to 6 carbon atoms, linear or branched alkyloxy of 1 to 6 carbon atoms, cycloalkyloxy of 5 to 10 carbon atoms, phenyl, naphthyl, anthryl, styryl, phenoxy, tolyloxy, benzyloxy, and phenethyloxy. These substituents may be further substituted.

Specific examples of the "aryloxy" in the "substituted or unsubstituted aryloxy" represented by $R_1$ to $R_{10}$ or $Ar_1$ to $Ar_5$ in general formulae (1) to (3) include phenoxy, biphenylyloxy, terphenylyloxy, naphthyloxy, anthryloxy, phenanthryloxy, fluorenyloxy, indenyloxy, and pyrenyloxy.

Specific examples of the "substituent" in the "substituted aryloxy" represented by $R_1$ to $R_{10}$ or $Ar_1$ to $Ar_5$ in general formulae (1) to (3) include a deuterium atom, a fluorine atom, a chlorine atom, cyano, trifluoromethyl, nitro, linear or branched alkyl of 1 to 6 carbon atoms, cycloalkyl of 5 to 10 carbon atoms, linear or branched alkyloxy of 1 to 6 carbon atoms, cycloalkyloxy of 5 to 10 carbon atoms, phenyl, naphthyl, anthryl, styryl, phenoxy, tolyloxy, benzyloxy, and phenethyloxy. These substituents may be further substituted.

Specific examples of the "divalent group of an aromatic hydrocarbon group", the "divalent group of an aromatic heterocyclic ring", or the "divalent group of a condensed polycyclic aromatic group" in the "divalent group of a substituted or unsubstituted aromatic hydrocarbon group", the "divalent group of a substituted or unsubstituted aromatic heterocyclic ring", or the "divalent group of a substituted or unsubstituted condensed polycyclic aromatic group" represented by B or C in general formulae (2) to (3) include phenylene, biphenylene, terphenylene, tetrakisphenylene, naphthylene, anthrylene, phenanthrylene, fluorenylene, phenanthrolylene, indenylene, pyrenylene, acenaphthenylene, fluoranthenylene, triphenylenylene, pyridinylene, pyrimidinylene, quinolylene, isoquinolylene, indolylene, carbazolylene, quinoxalylene, benzoimidazolylene, pyrazolylene, naphthyridinylene, phenanthrolinylene, acridinylene, thienylene, benzothienylene, and dibenzothienylene.

Specific examples of the "substituent" in the "divalent group of a substituted aromatic hydrocarbon group", the "divalent group of a substituted aromatic heterocyclic ring", or the "divalent group of a substituted condensed polycyclic aromatic group" represented by B or C in general formulae (2) to (3) include a deuterium atom, a fluorine atom, a chlorine atom, cyano, trifluoromethyl, nitro, linear or branched alkyl of 1 to 6 carbon atoms, cycloalkyl of 5 to 10 carbon atoms, linear or branched alkenyl of 2 to 6 carbon atoms, linear or branched alkyloxy of 1 to 6 carbon atoms, cycloalkyloxy of 5 to 10 carbon atoms, phenyl, naphthyl, anthryl, styryl, phenoxy, tolyloxy, benzyloxy, and phenethyloxy. These substituents may be further substituted.

In the present invention, at least one of $R_1$ to $R_{10}$, or at least one of the substituents of $Ar_1$ to $Ar_5$, B or C in the general formulae (1) to (3) is preferably a deuterium atom, or a substituent that contains a deuterium atom. The deuterium atom, or the substituent containing a deuterium atom should be contained in as large numbers as possible. For example, in general formula (1), it is preferable that a deuterium atom replaces all of $R_1$ when n is 0 and $r_1$ is 4, all of $R_1$ when n is 1 and $r_1$ is 3, all of $R_2$ when $r_2$ is 3, all of $R_3$ when $r_3$ is 3, all of $R_4$ when $r_4$ is 4, all of $R_5$ when n is 1 and $r_5$ is 4, or all of $R_6$ when n is 1 and $r_6$ is 3. Further, for example, it is preferable in general formula (2) that a deuterium atom replaces all of $R_7$ when $r_7$ is 4, or all of $R_8$ when $r_8$ is 3. Further, for example, it is preferable in general formula (3) that a deuterium atom replaces all of $R_9$ when $r_9$ is 3, or all of $R_{10}$ when $r_{10}$ is 3. Further, in $Ar_1$ to $Ar_5$ in general formulae (1), (2), or (3), it is preferable that $Ar_1$ to $Ar_5$ be an aromatic hydrocarbon group, an aromatic heterocyclic group, or a condensed polycyclic aromatic group in which all of the hydrogen atoms are substituted with deuterium atoms, or in which all the substituents are substituted with deuterium atoms. Further, in B and C in general formulae (2) and (3), it is preferable that B and C be an aromatic hydrocarbon group, an aromatic heterocyclic group, or a condensed polycyclic aromatic group in which all of the hydrogen atoms are substituted with deuterium atoms, or in which all the substituents are substituted with deuterium atoms.

Among the compounds of the general formula (1) having a carbazole ring structure, the compounds of the following general formula (1') are preferably used for an organic EL device.

[Chemical Formula 9]

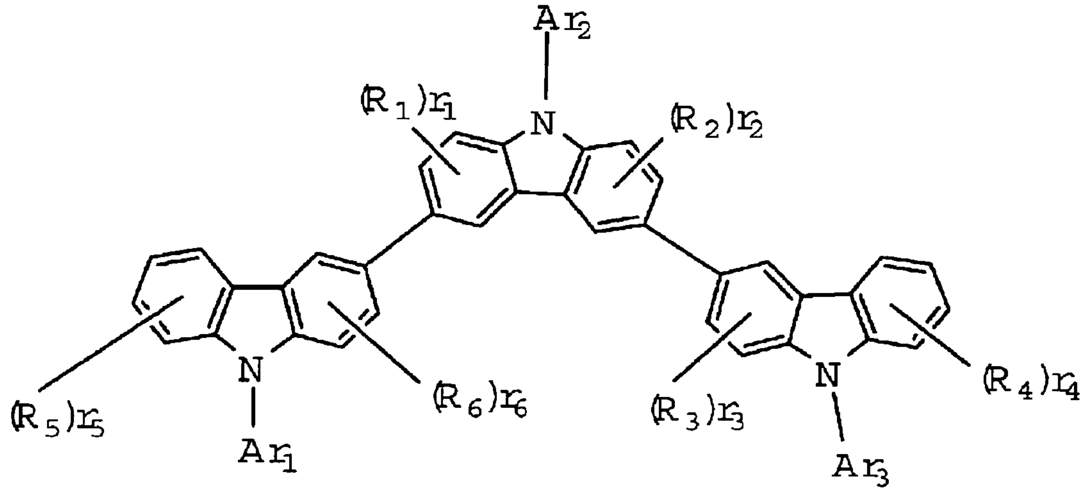

(1')

In the formula, $R_1$, $R_2$, $R_3$, $R_4$, $R_5$, and $R_6$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. $r_4$ and $r_5$ may be the same or different, and represent 0 or an integer of 1 to 4. $r_1$, $r_2$, $r_3$, and $r_6$ may be the same or different, and represent 0 or an integer of 1 to 3. $Ar_1$, $Ar_2$, and $Ar_3$ may be the same or different, and represent a substituted or unsubstituted aromatic hydrocarbon group, a substituted or unsubstituted aromatic heterocyclic group, or a substituted or unsubstituted condensed polycyclic aromatic group. At least one of $R_1$ to $R_6$, or at least one of the substituents of $Ar_1$ to $Ar_3$ is a deuterium atom, or a substituent that contains a deuterium atom.

The compounds of general formula (1) having a carbazole ring structure used for the organic EL device of the present invention are highly capable of confining triplet excitons, and have superior electron blocking ability and heat resistance, and a stable thin-film state.

The compounds of general formula (1) having a carbazole ring structure used for the organic EL device of the present invention may be used as a constituent material of the hole injection layer and/or the hole transport layer of the organic EL device, particularly a phosphorescent organic EL device. With the compounds having high hole injectability, high mobility, high $T_1$ value, and high electron stability, the triplet excitons generated in the light emitting layer containing the phosphorescent light-emitting material can be confined, and the probability of hole-electron recombination can be improved. This improves the luminous efficiency, and lowers driving voltage and thus improves the durability of the organic EL device.

The compounds of general formula (1) having a carbazole ring structure used for the organic EL device of the present invention may be used as a constituent material of the electron blocking layer of the organic EL device, particularly a phosphorescent organic EL device. With the material having high triplet exciton confining capability and excellent hole transportability with high stability in the thin-film state, the driving voltage lowers and the current resistance improves while maintaining high luminous efficiency. As a result, the maximum emission luminance of the organic EL device improves.

The compounds of general formula (1) having a carbazole ring structure used for the organic EL device of the present invention also may be used as a constituent material of the light emitting layer of the organic EL device, particularly a phosphorescent organic EL device. The compounds have excellent hole transportability and a wide band gap, and can thus be used as the host material of the light emitting layer to form the light emitting layer by carrying a phosphorescent material called a dopant. In this way, an organic EL device can be realized that has a low driving voltage and improved luminous efficiency.

The organic EL device of the present invention uses the compound having a carbazole ring structure, wherein the compound has high hole mobility and excellent triplet exciton confining capability while having a stable thin-film state. In this way, high efficiency and high durability are realized.

## Advantage of the Invention

The compound having a carbazole ring structure used for the organic EL device of the present invention is useful as a constituent material of the hole injection layer, the hole transport layer, and the electron blocking layer of the organic EL device, particularly a phosphorescent organic EL device. The compound has excellent triplet exciton confining capability, and excels in heat resistance while having a stable thin-film state. The organic EL device of the present invention has high luminous efficiency and high power efficiency, and can thus lower the actual driving voltage of the device. Further, the turn on voltage can be lowered to improve durability.

## Brief Description of the Drawings

FIG. 1 is a 1H-NMR chart of the compound of Example 1 of the present invention (Compound 11).
FIG. 2 is a 1H-NMR chart of the compound of Example 2 of the present invention (Compound 12).
FIG. 3 is a 1H-NMR chart of the compound of Example 3 of the present invention (Compound 54).
FIG. 4 is a diagram representing the configuration of the organic EL devices of Examples 7 and 8 and Comparative Examples 1 and 2.

## Mode for Carrying Out the Invention

The compounds having a carbazole ring structure used in the present invention are novel compounds, and may be synthesized with deuterated material by using known methods (see, for example, Patent Document 3), or by using, for example, the following method. First, a monobromocarbazole such as 3-bromo-9-arylcarbazole, or a dibromocarbazole such as 3,6-dibromo-9-arylcarbazole is synthesized by the bromination of a carbazole substituted with an aryl group at the corresponding ninth position, using, for example, N-bromosuccinimide (see, for example, Non-Patent Document 6). The boronic acid or borate synthesized by the reaction of the resulting monobromocarbazole with compounds such as pinacolborane or bis(pinacolato)diboron (see, for example, Non-Patent Document 7) can then be reacted with dibromocarbazole or monobromocarbazole in a cross-coupling reaction such as Suzuki coupling (see, for example, Non-Patent Document 8) to synthesize bis(N-aryl-9'H-carbazol-3'-yl)-9-aryl-9H-carbazole or (N-aryl-9'H-carbazol-3'-yl)-9H-carbazole. The (N-aryl-9'H-carbazol-3'-yl)-9-halogenoaryl-carbazole obtained by the condensation reaction (such as Ullmann reaction) of the (N-aryl-9'H-carbazol-3'-yl)-9H-carbazole with various dihalogenoarylenes can be reacted with 3-boronic acid or borate of 9-arylcarbazole in a cross-coupling reaction such as Suzuki coupling (see, for example, Non-Patent Document 8) to synthesize a compound having a carbazole ring structure.
A target compound having a deuterium atom-substituted carbazole ring structure can be synthesized by using the foregoing producing process with a raw material substituted with a deuterium atom at the corresponding position of a carbazole, an aryl, or the like.

The following presents specific examples of preferred compounds among the compounds of general formula (1) having a carbazole ring structure. The present invention, however, is not restricted to these compounds.

[Chemical Formula 15]

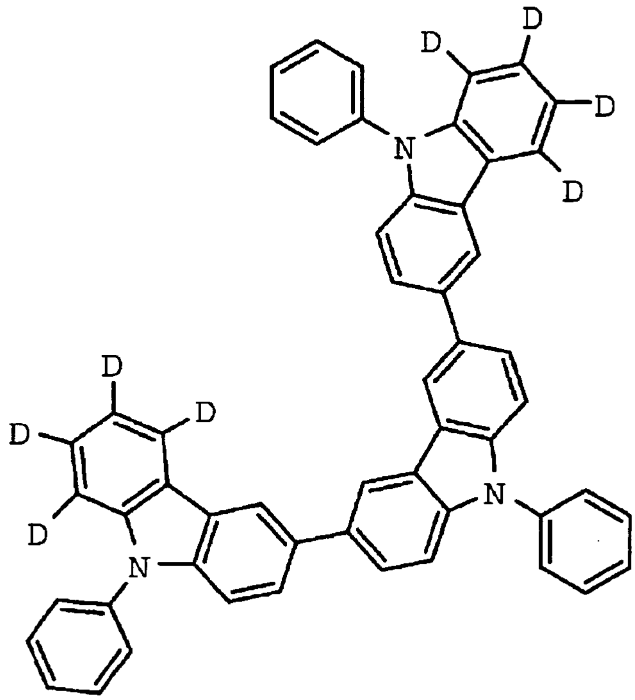

(Compound 5)

[Chemical Formula 16]

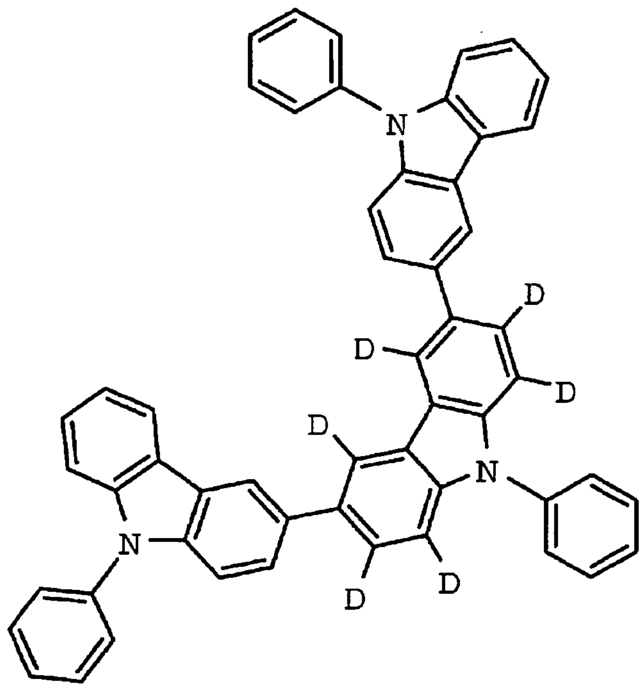

(Compound 6)

[Chemical Formula 17]

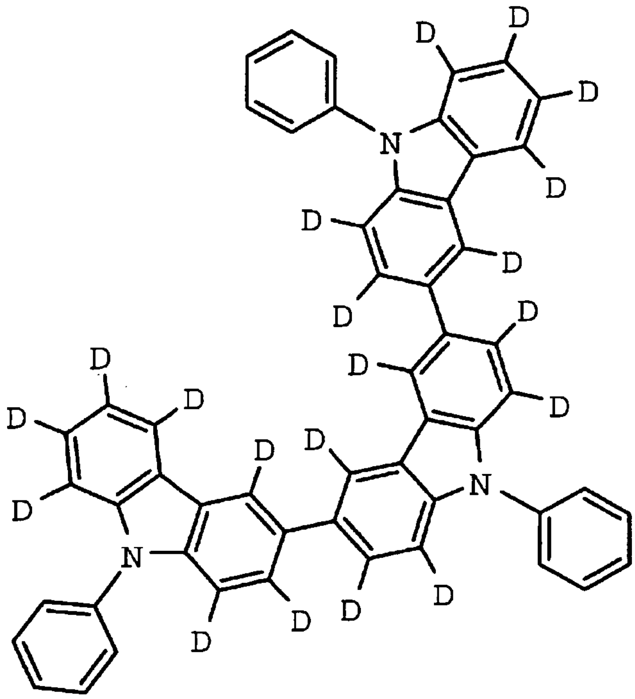

(Compound 7)

[Chemical Formula 18]

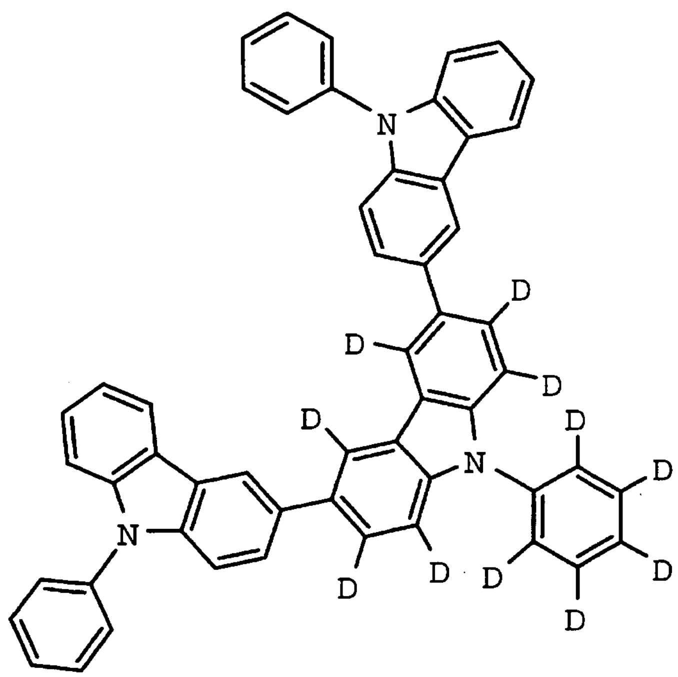

(Compound 8)

[Chemical Formula 19]

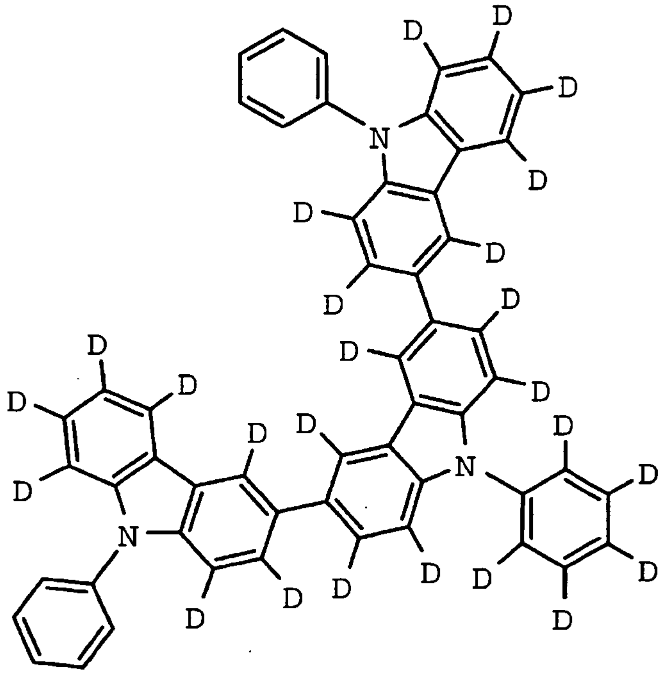

(Compound 9)

[Chemical Formula 20]

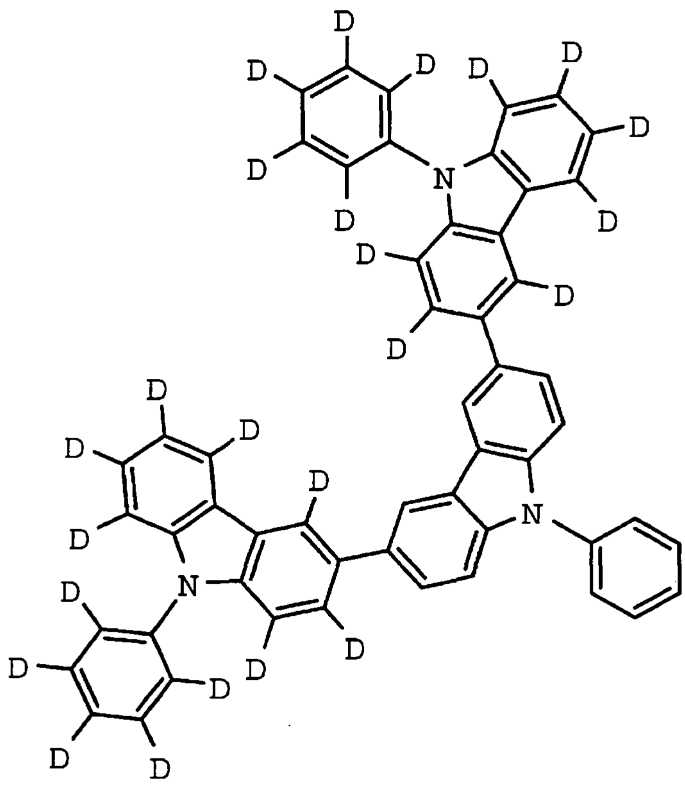

(Compound 10)

[Chemical Formula 21]

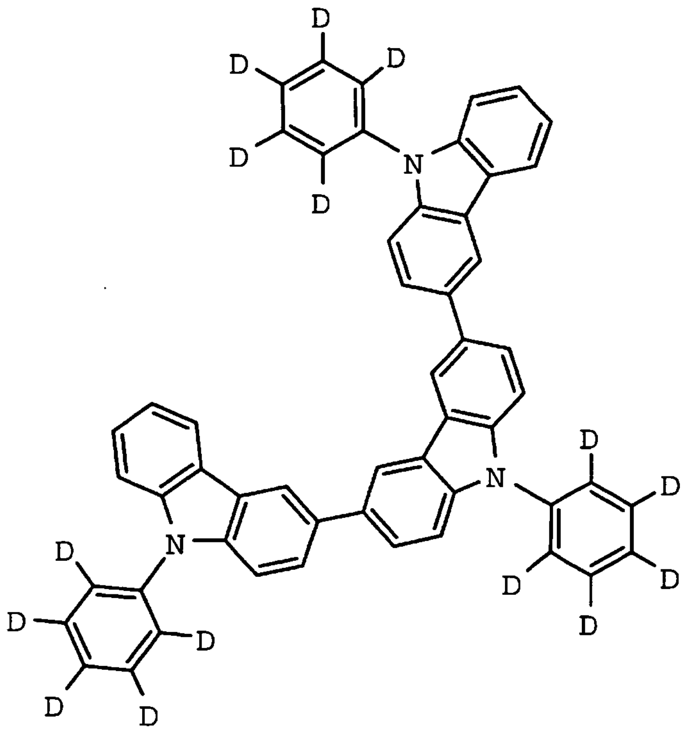

(Compound 11)

[Chemical Formula 22]

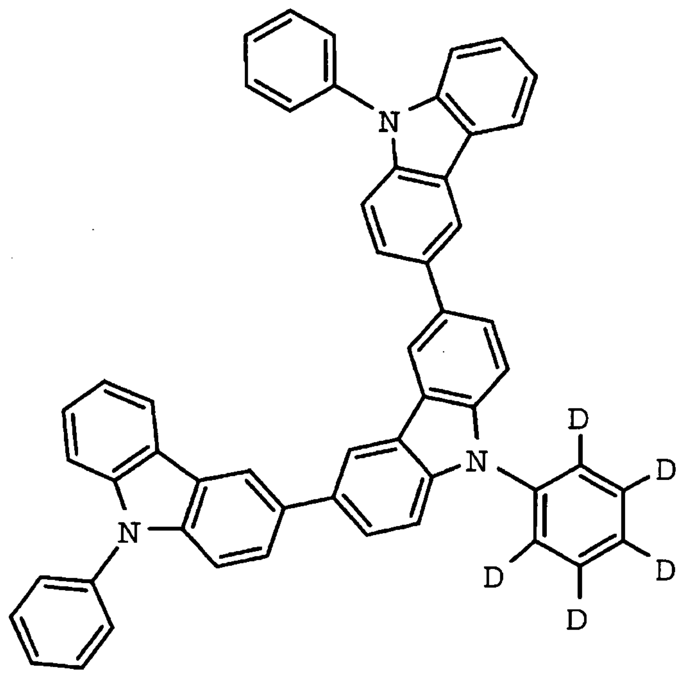

(Compound 12)

[Chemical Formula 23]

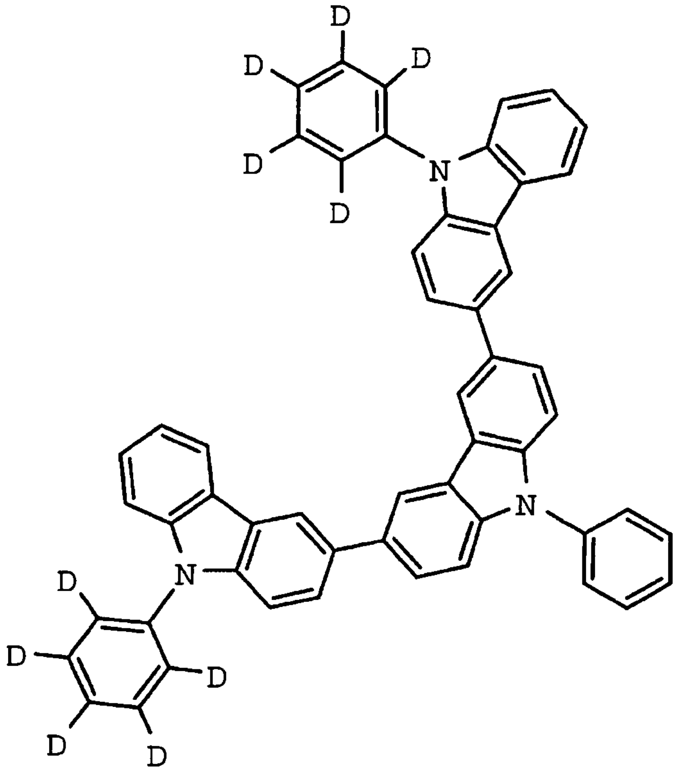

(Compound 13)

[Chemical Formula 24]

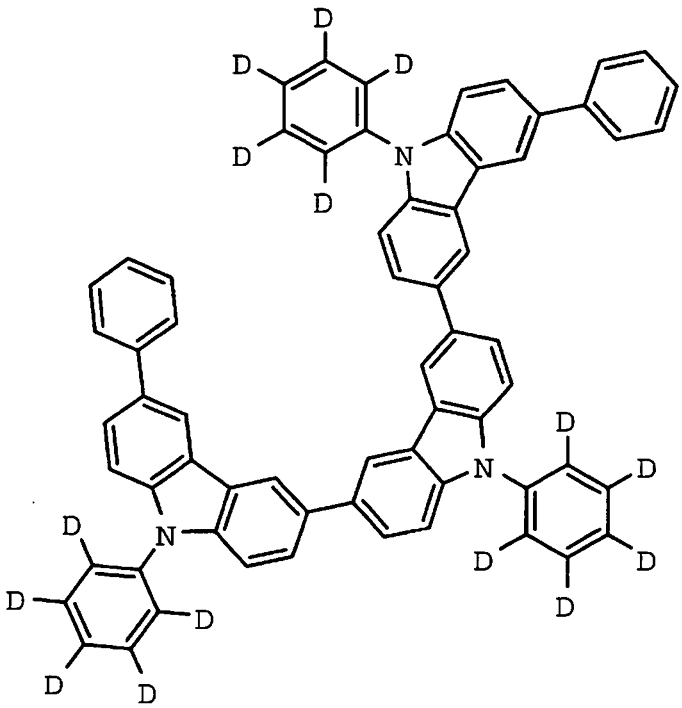

(Compound 14)

[Chemical Formula 25]

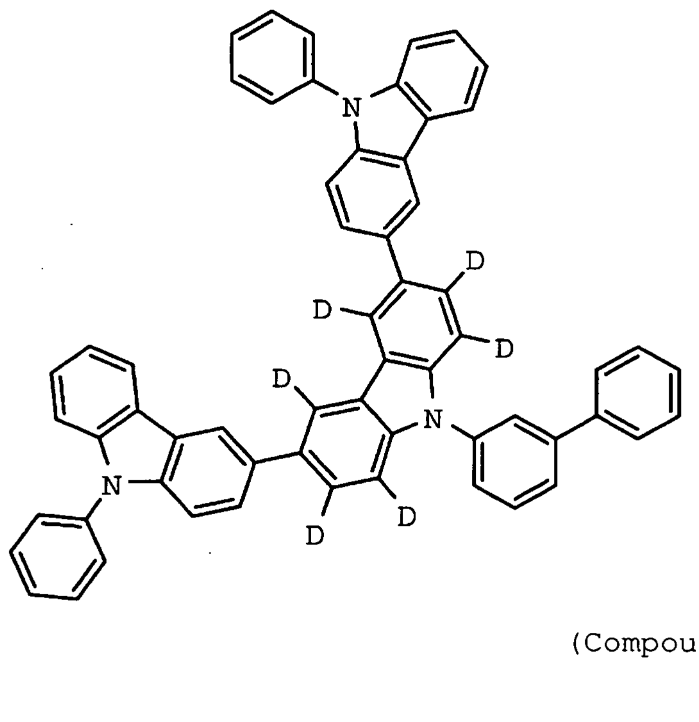

(Compound 15)

[Chemical Formula 26]

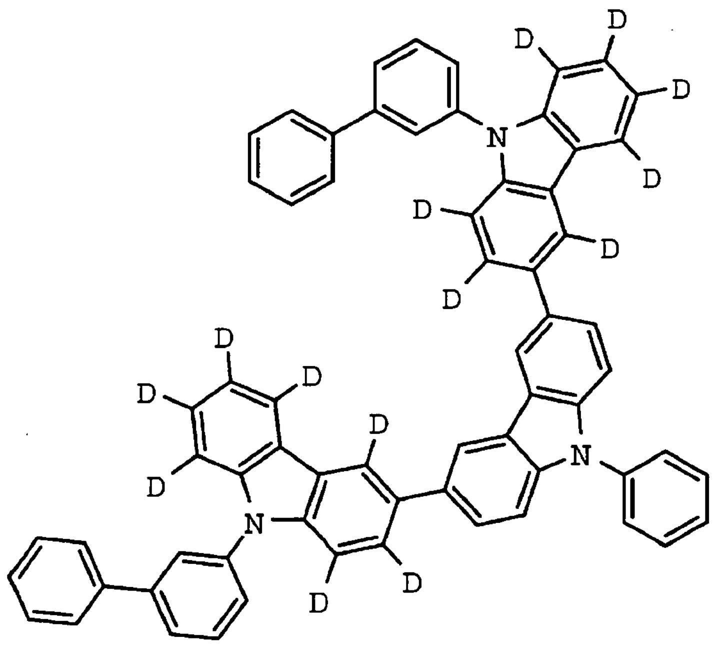

(Compound 16)

[Chemical Formula 27]

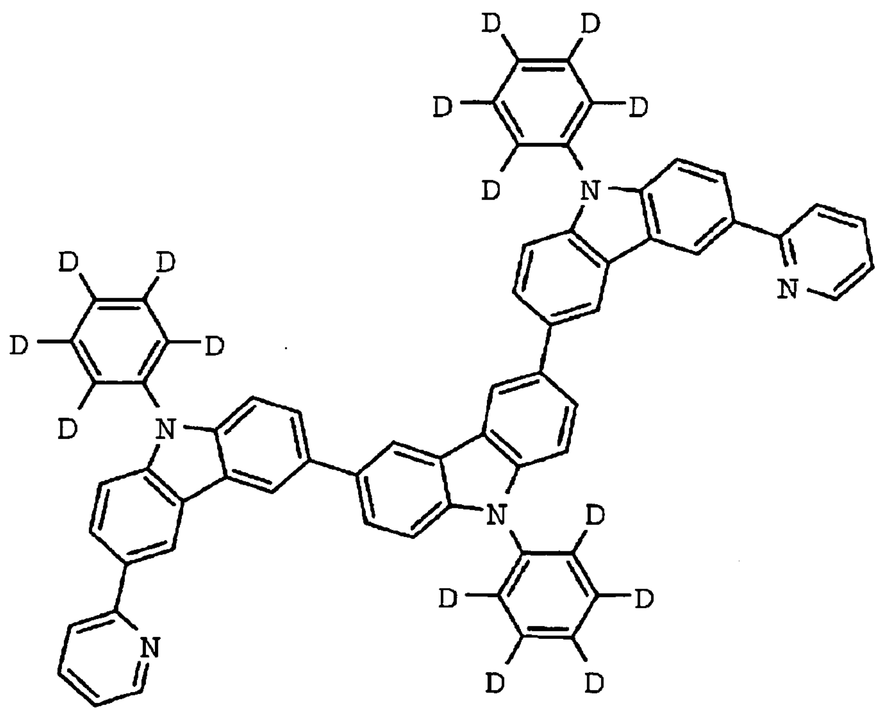

(Compound 17)

[Chemical Formula 28]

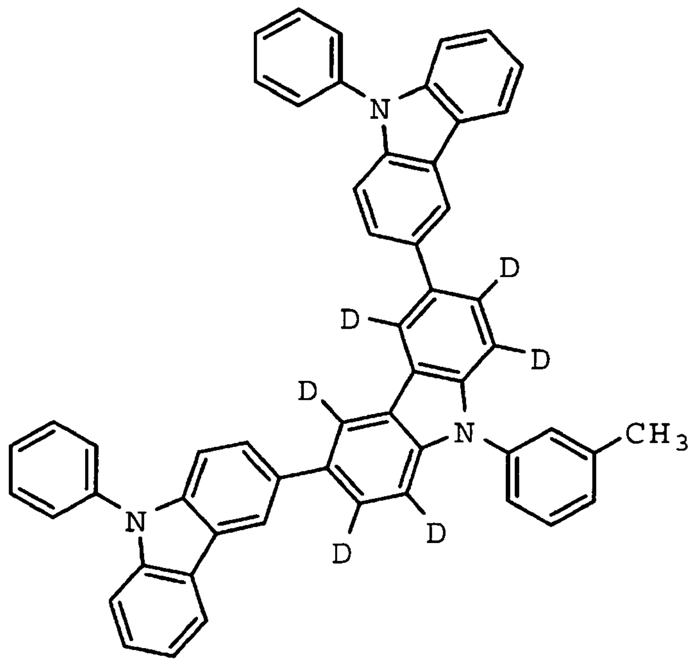

(Compound 18)

[Chemical Formula 29]

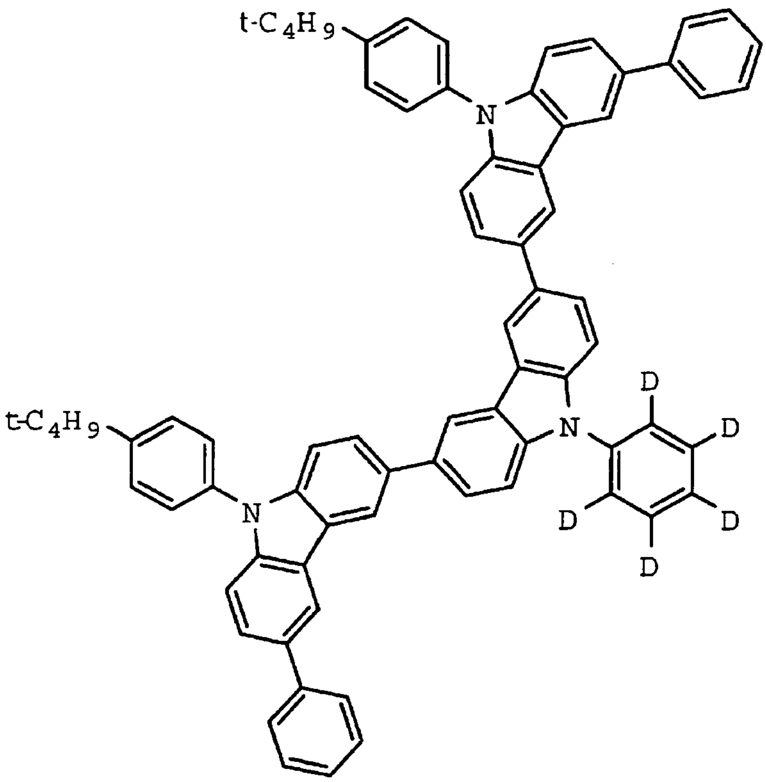

(Compound 19)

[Chemical Formula 30]

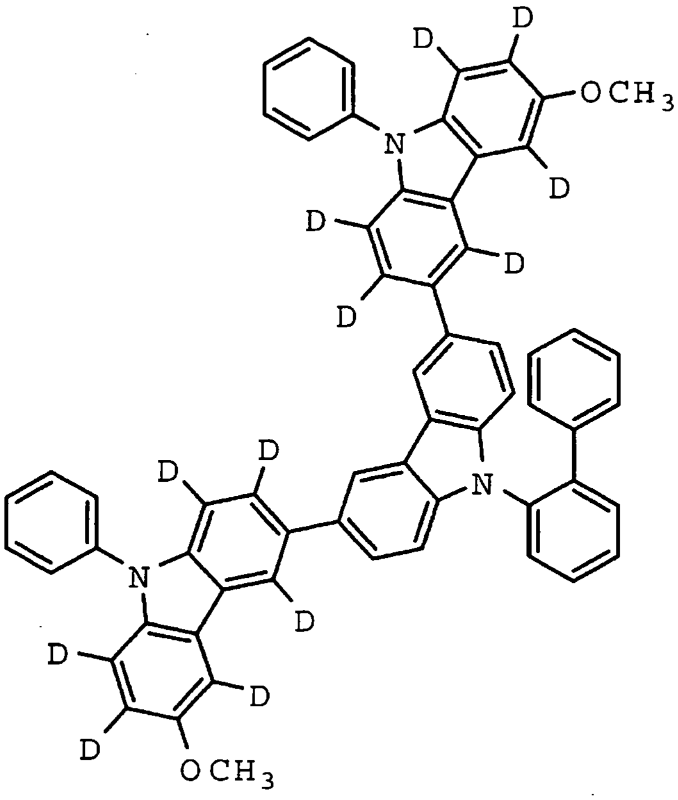

(Compound 20)

[Chemical Formula 31]

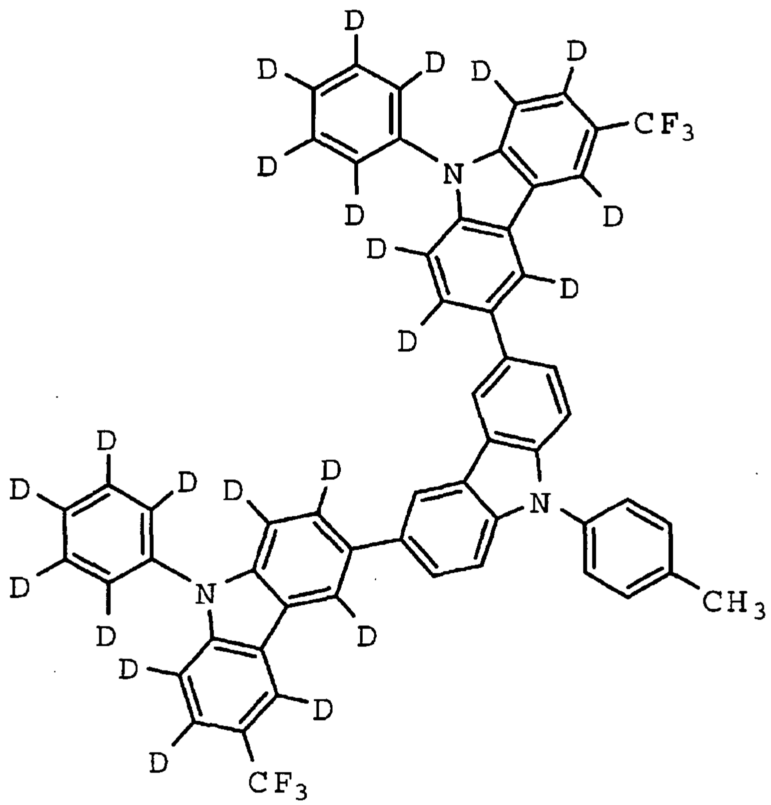

(Compound 21)

[Chemical Formula 32]

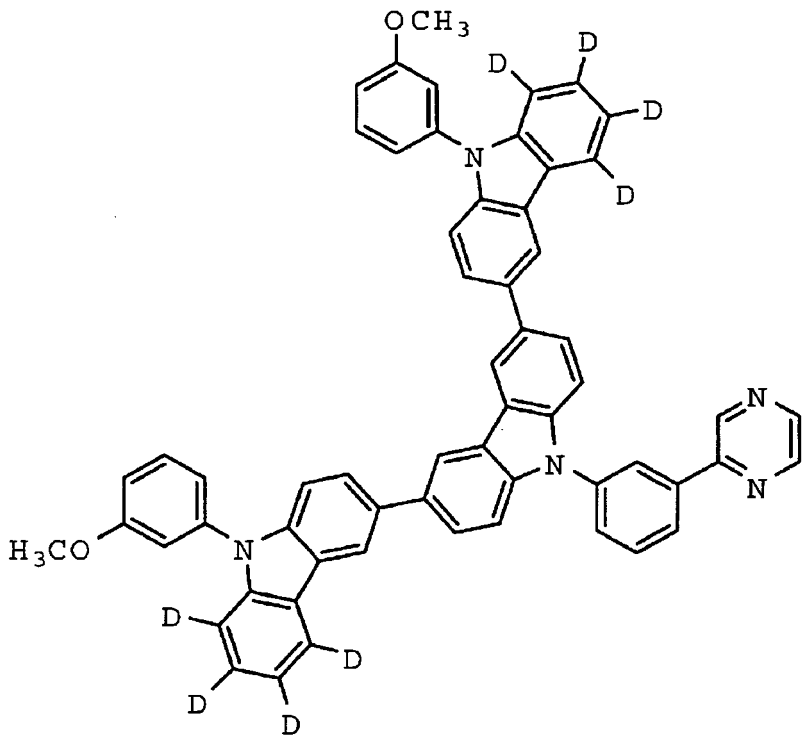

(Compound 22)

[Chemical Formula 33]

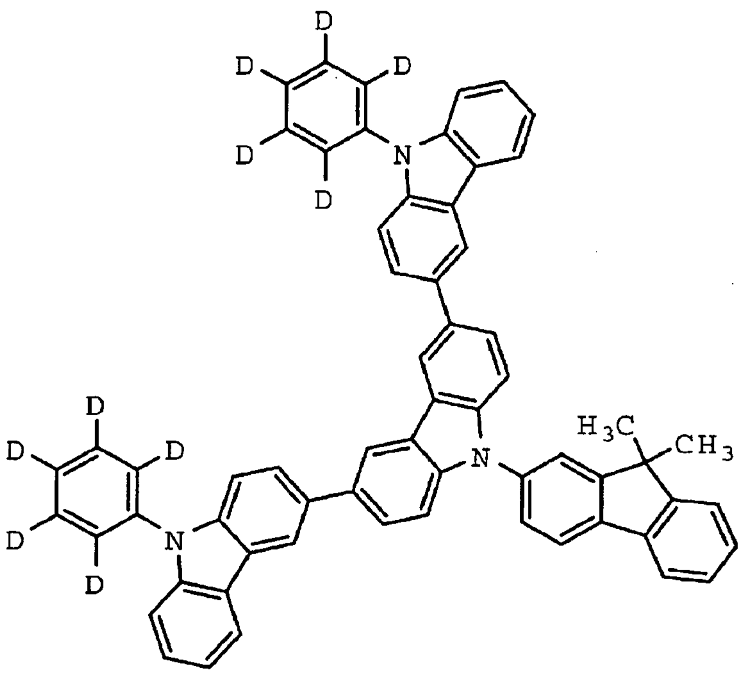

(Compound 23)

[Chemical Formula 34]

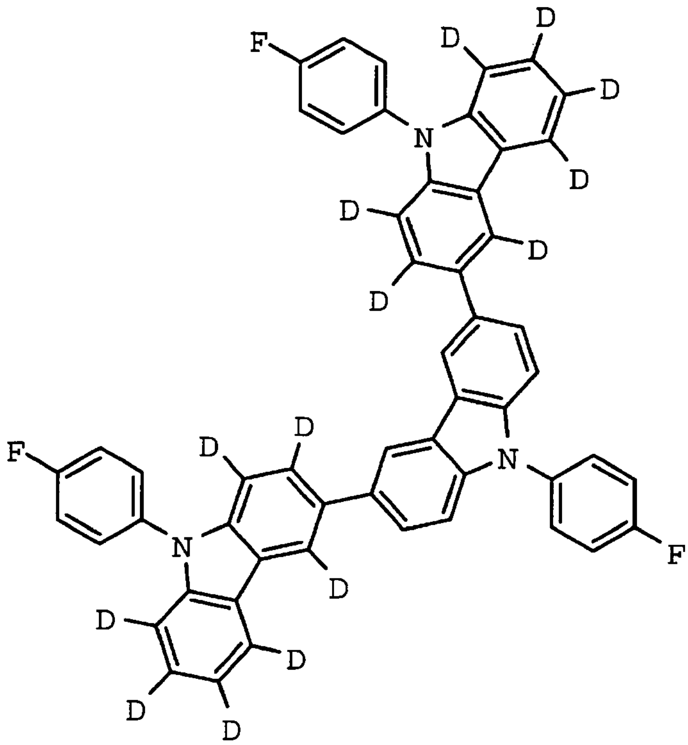

(Compound 24)

[Chemical Formula 35]

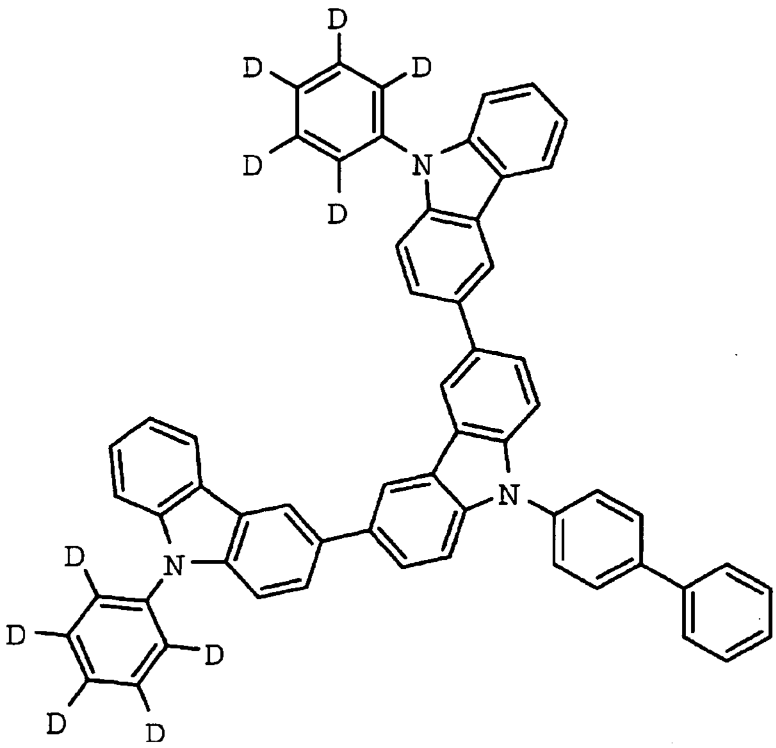

(Compound 25)

[Chemical Formula 36]

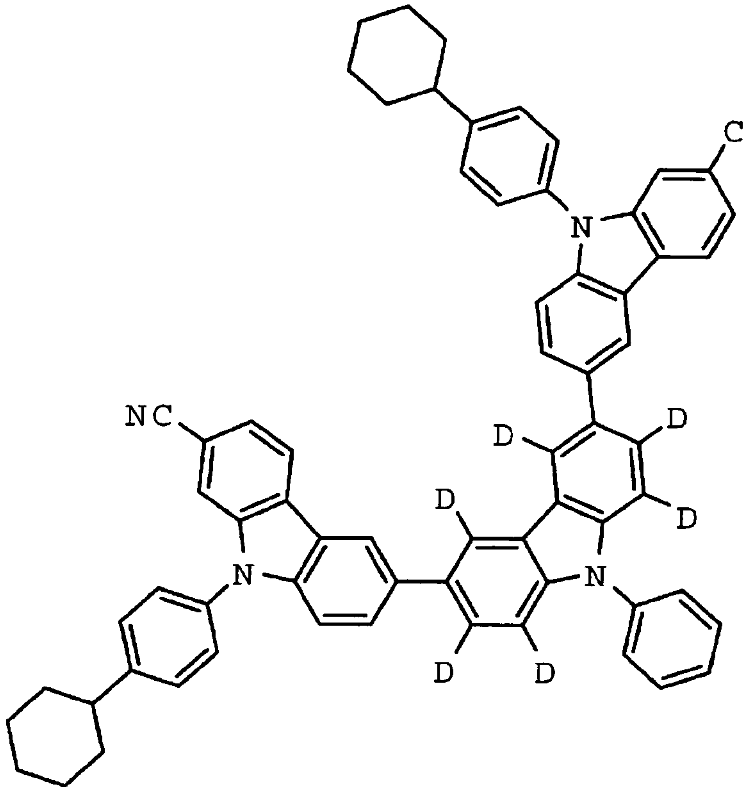

(Compound 26)

[Chemical Formula 37]

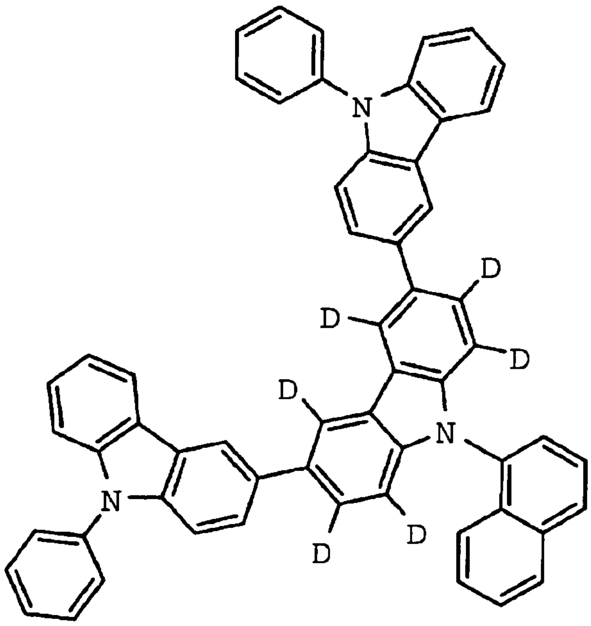

(Compound 27)

[Chemical Formula 38]

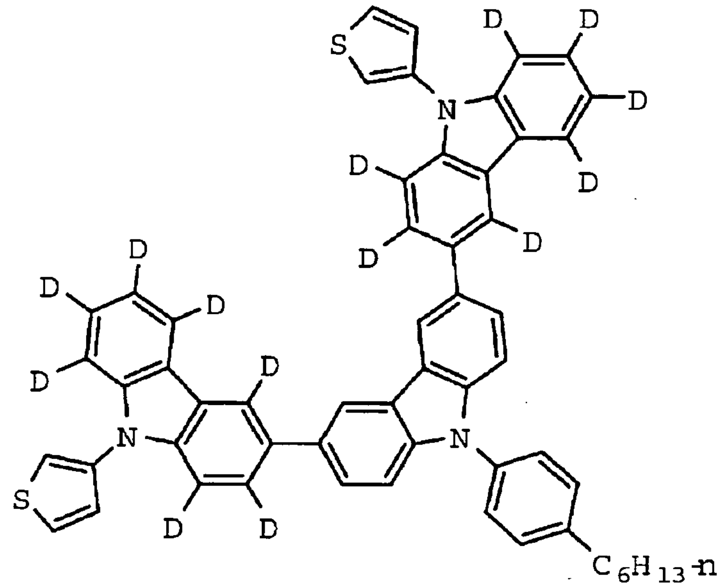

(Compound 28)

[Chemical Formula 39]

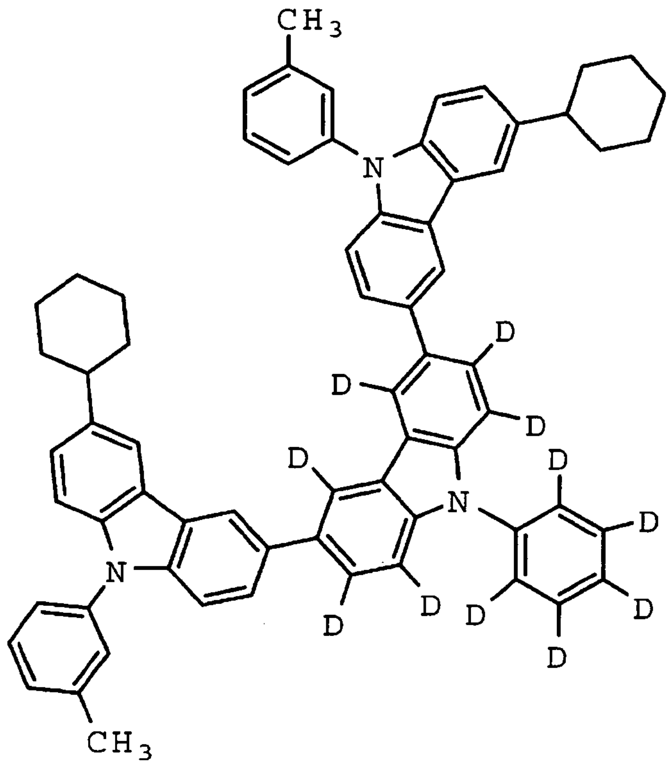

(Compound 29)

[Chemical Formula 40]

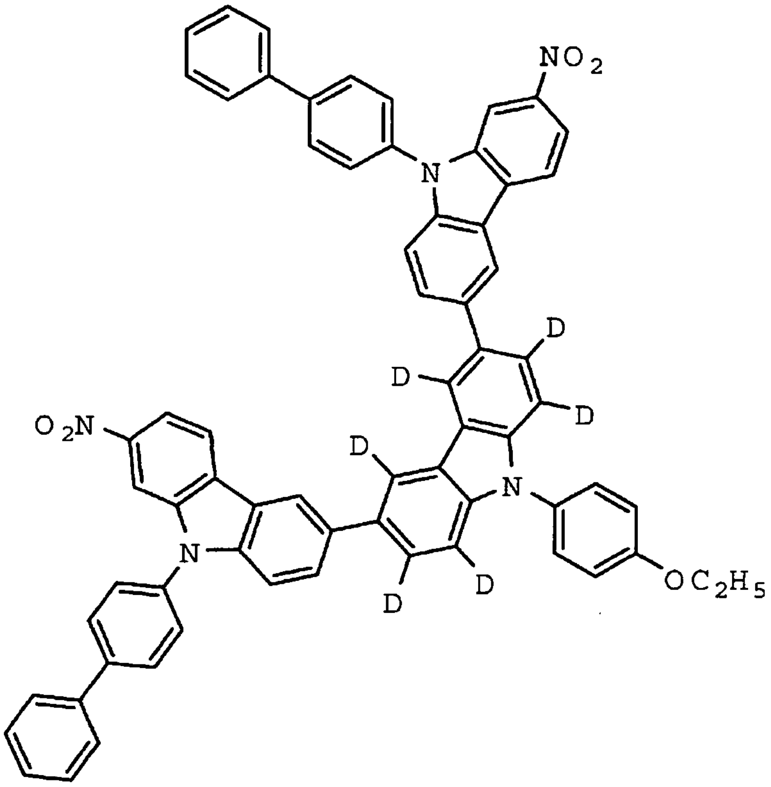

(Compound 30)

[Chemical Formula 41]

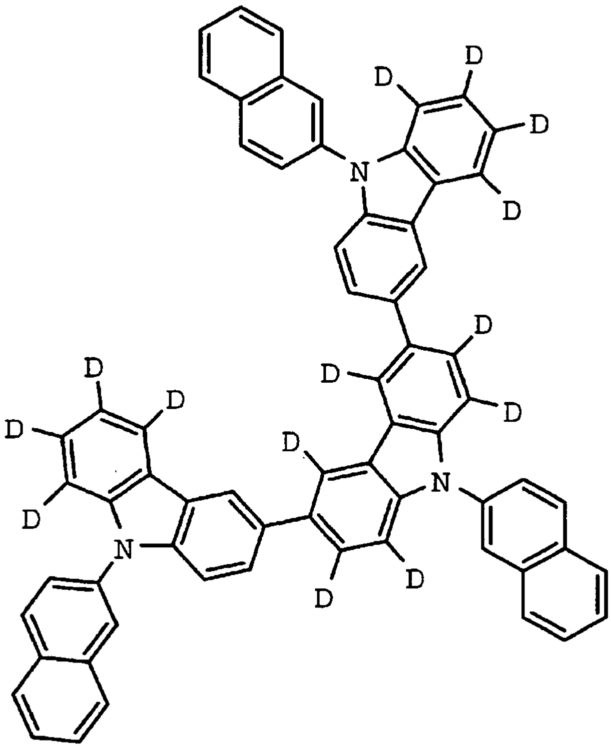

(Compound 31)

[Chemical Formula 42]

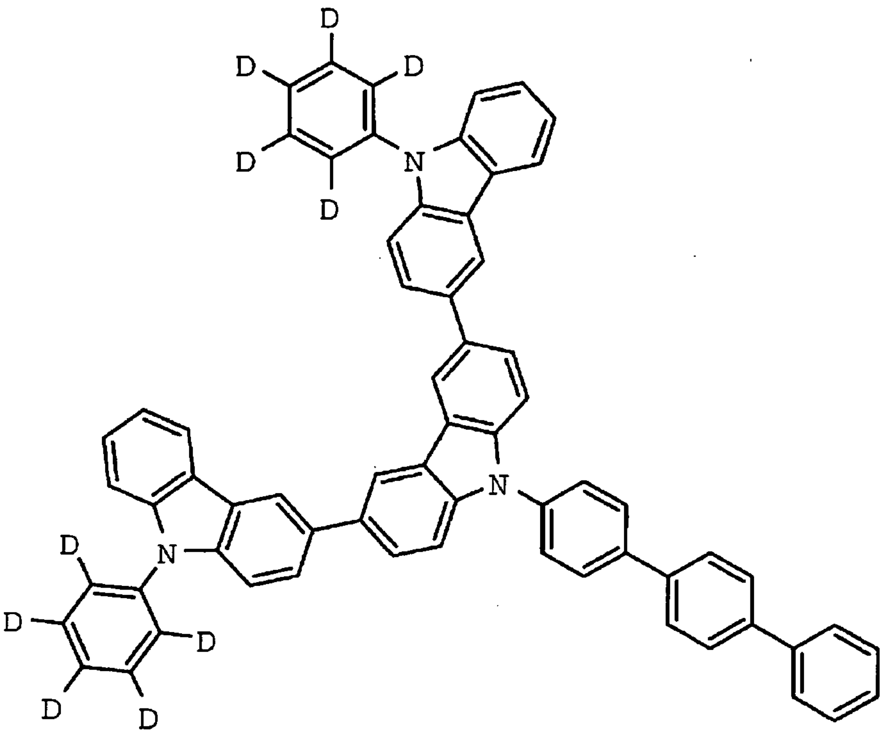

(Compound 32)

[Chemical Formula 43]

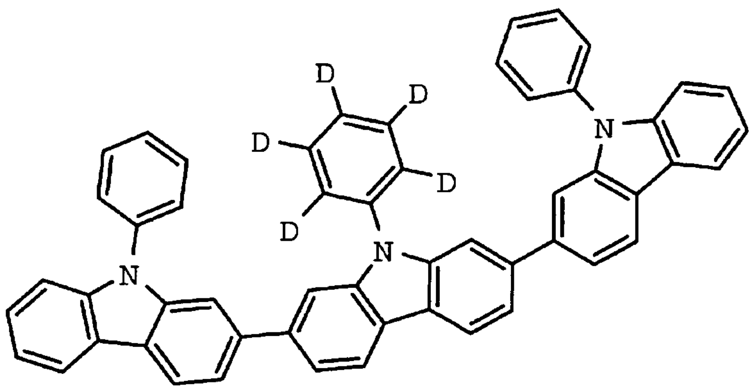

(Compound 33)

[Chemical Formula 44]

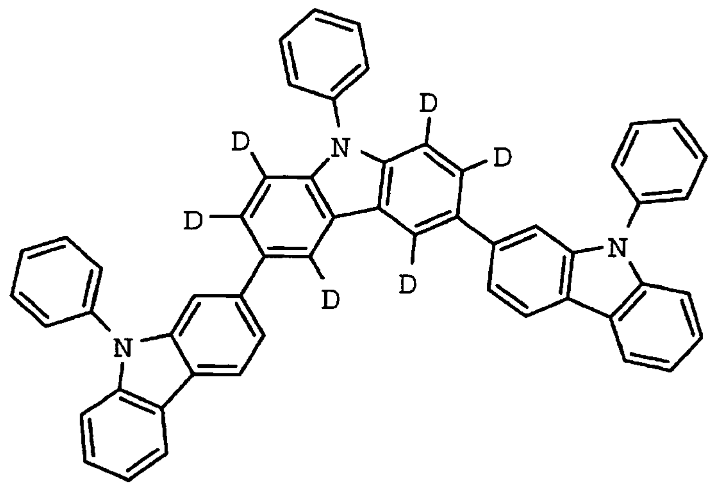

(Compound 34)

[Chemical Formula 45]

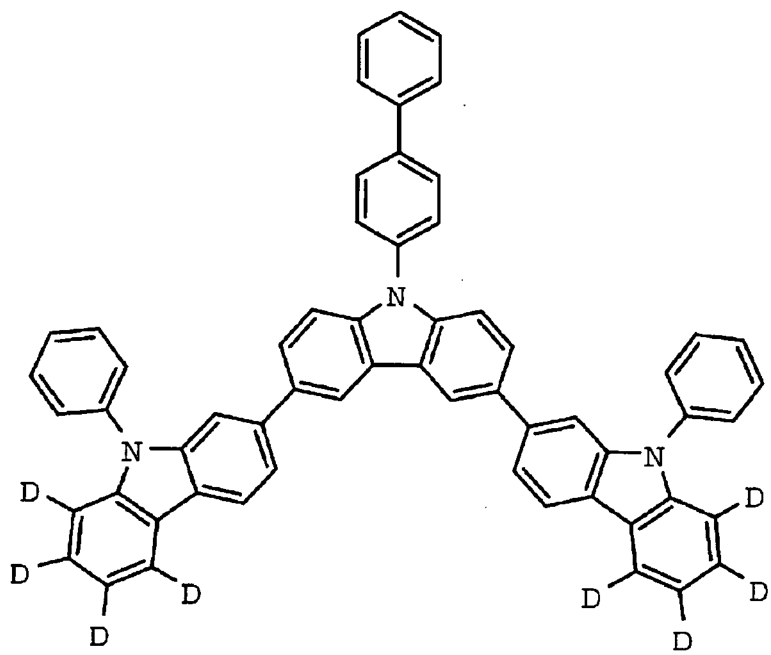

(Compound 35)

[Chemical Formula 46]

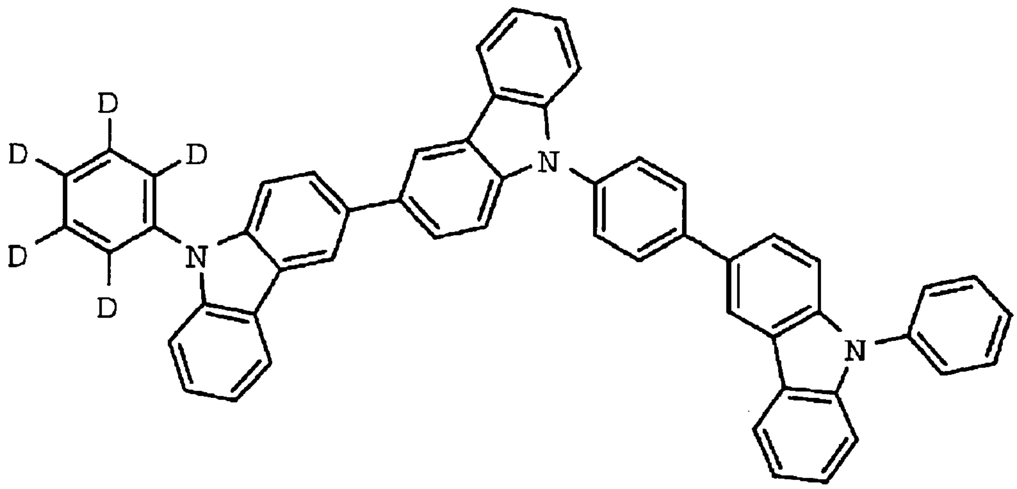

(Compound 36)

[Chemical Formula 47]

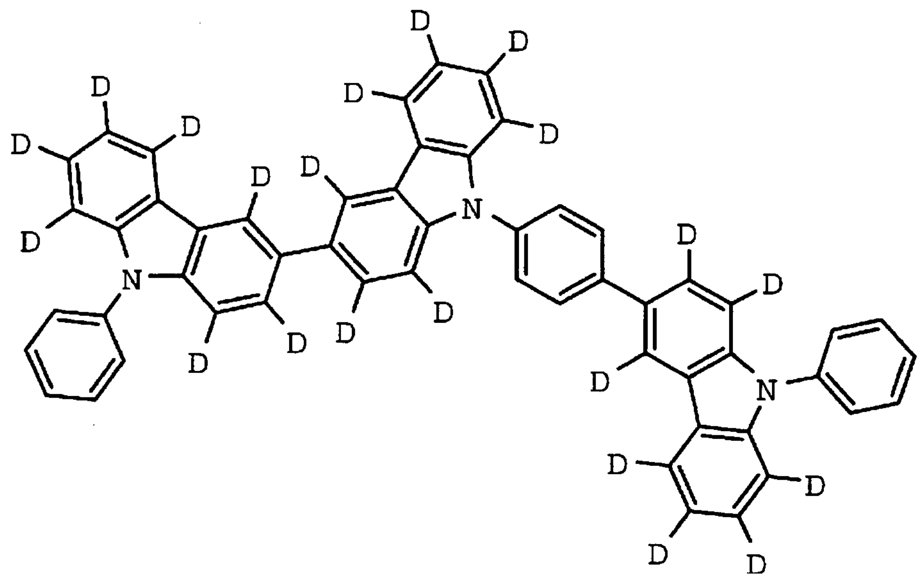

(Compound 37)

[Chemical Formula 48]

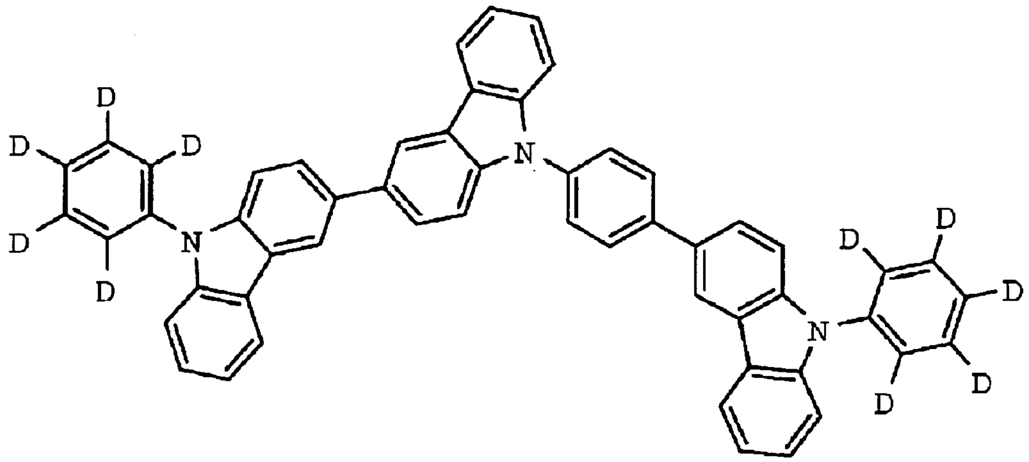

(Compound 38)

[Chemical Formula 49]

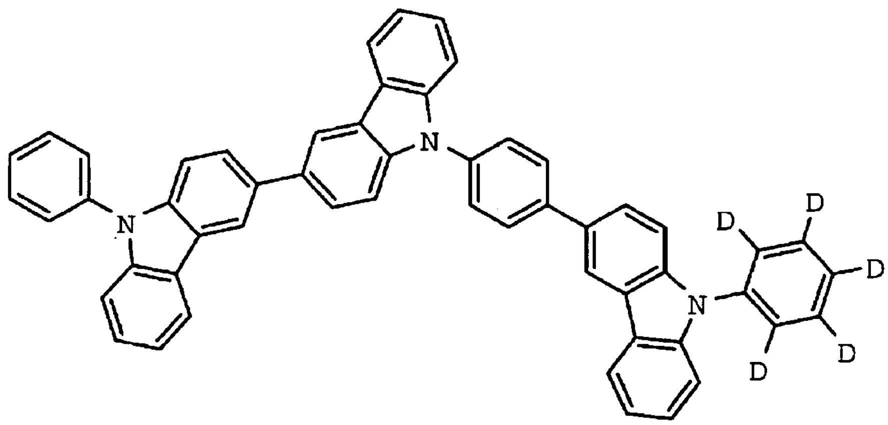

(Compound 39)

[Chemical Formula 50]

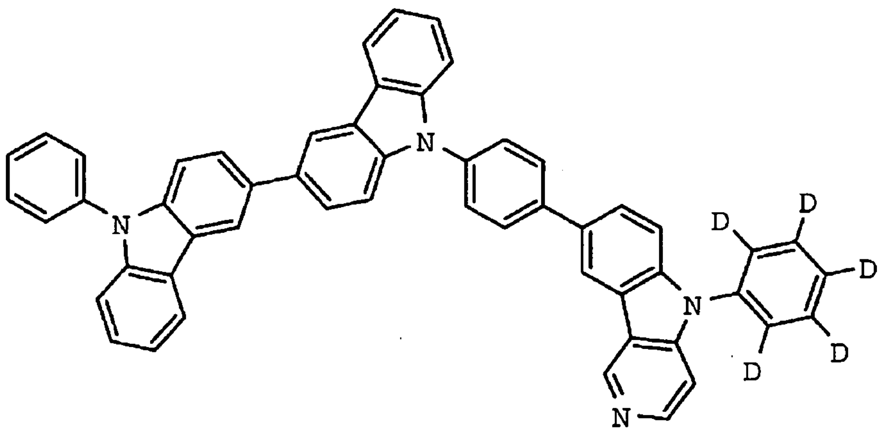

(Compound 40)

[Chemical Formula 51]

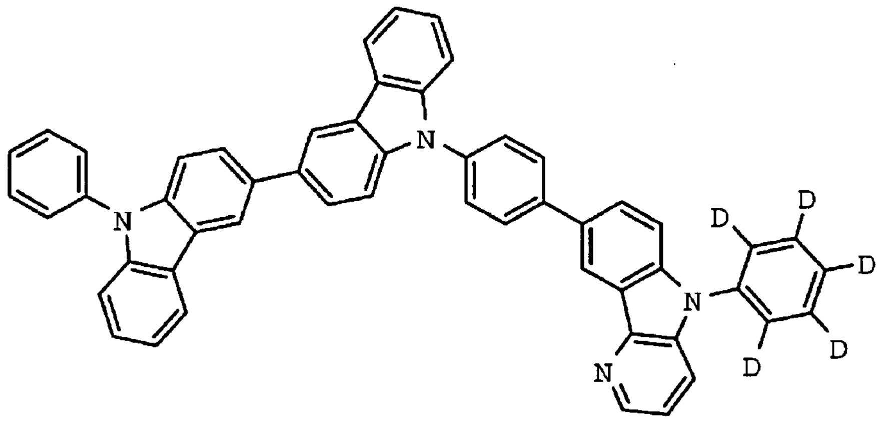

(Compound 41)

[Chemical Formula 52]

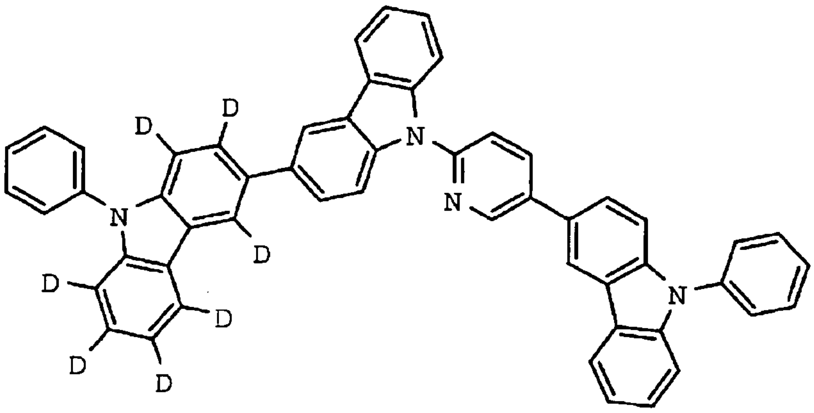

(Compound 42)

[Chemical Formula 53]

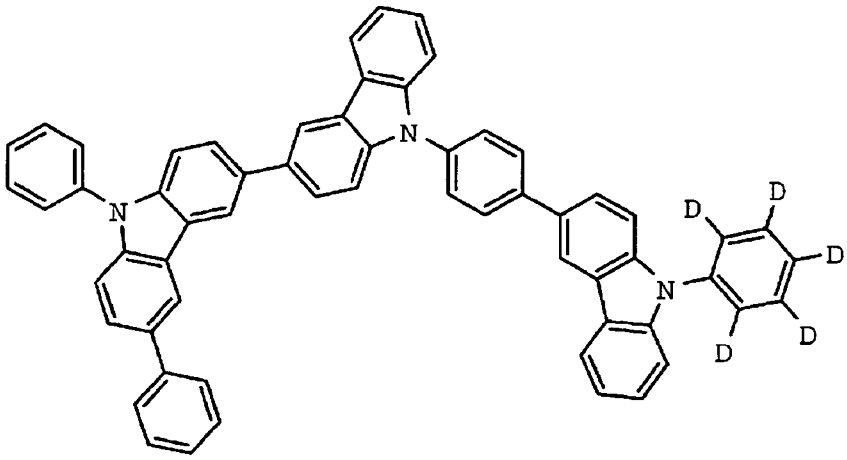

(Compound 43)

[Chemical Formula 54]

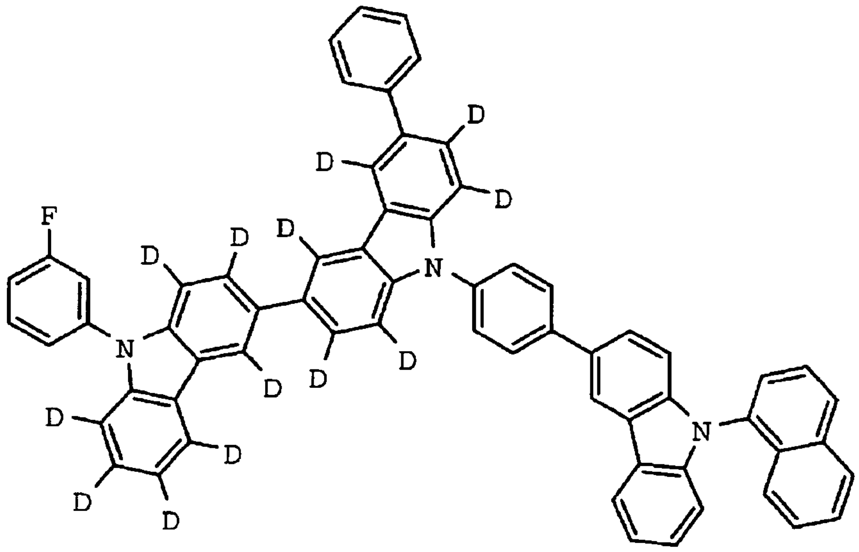

(Compound 44)

[Chemical Formula 55]

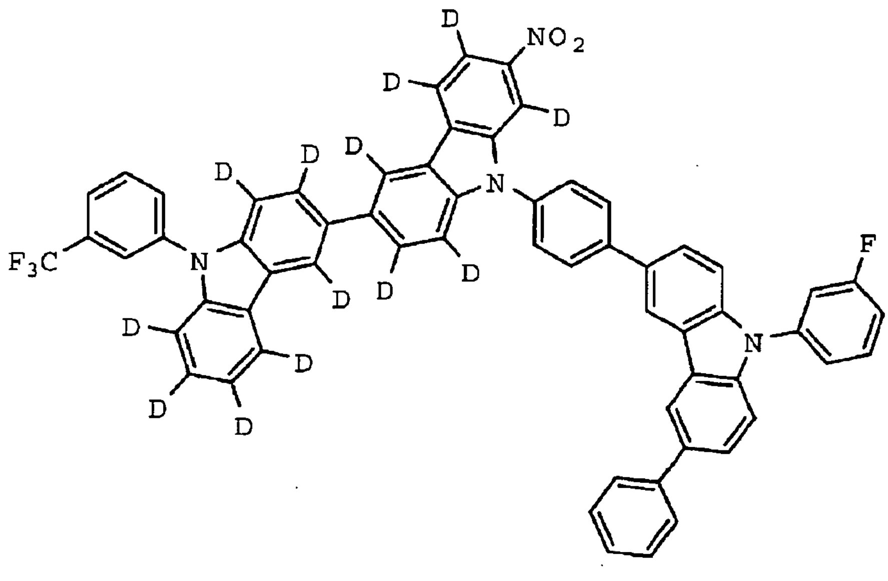

(Compound 45)

[Chemical Formula 56]

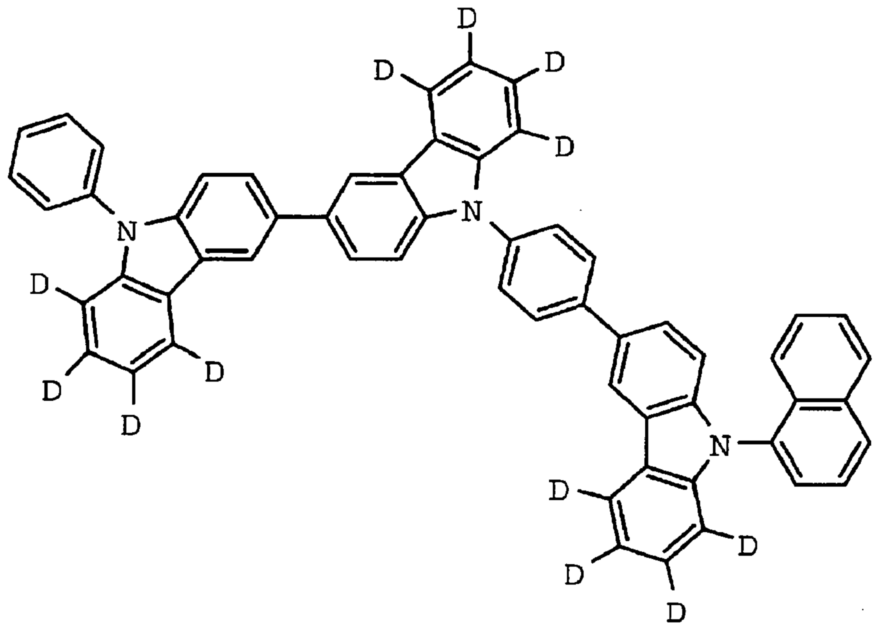

(Compound 46)

[Chemical Formula 57]

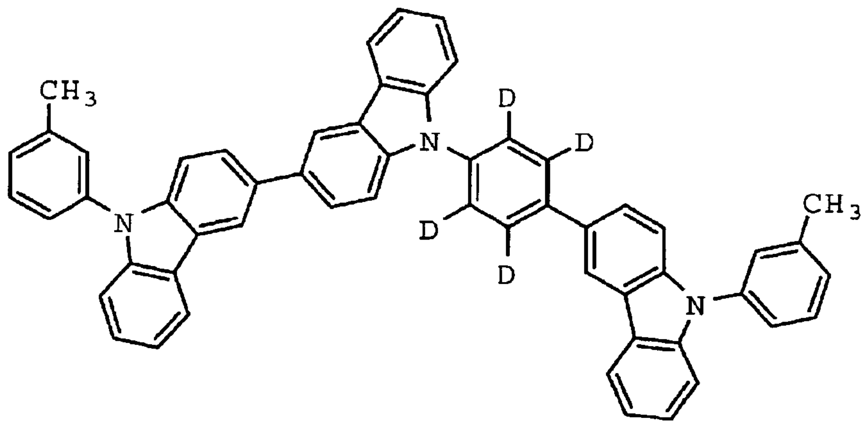

(Compound 47)

[Chemical Formula 58]

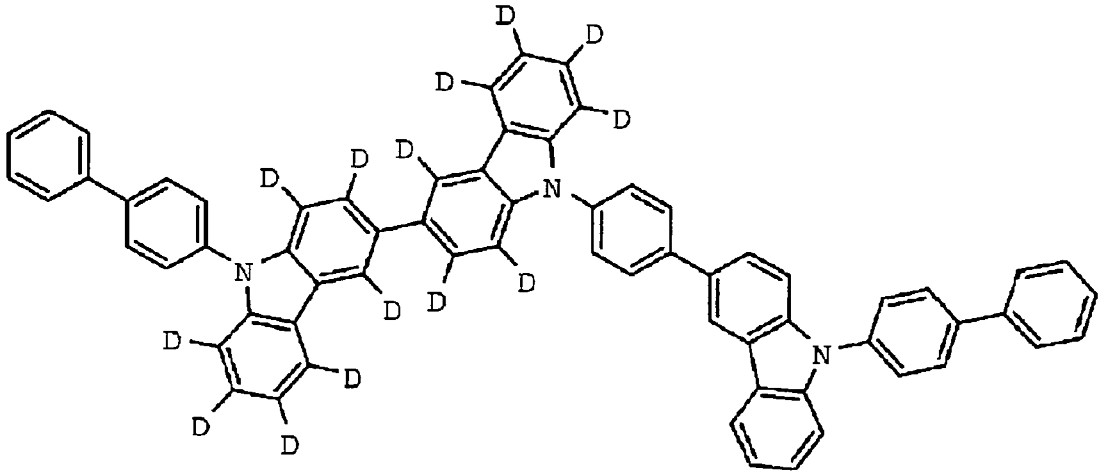

(Compound 48)

[Chemical Formula 59]

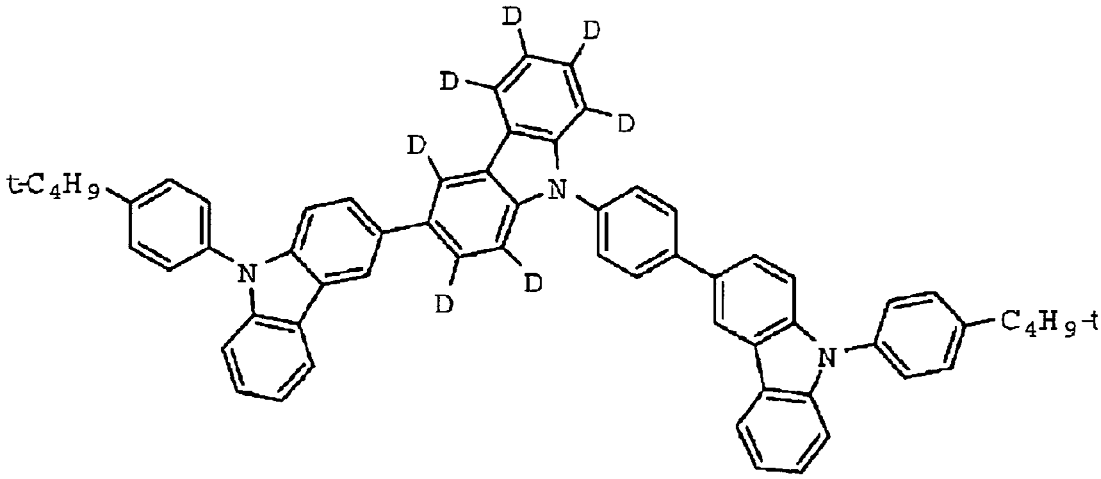

(Compound 49)

[Chemical Formula 60]

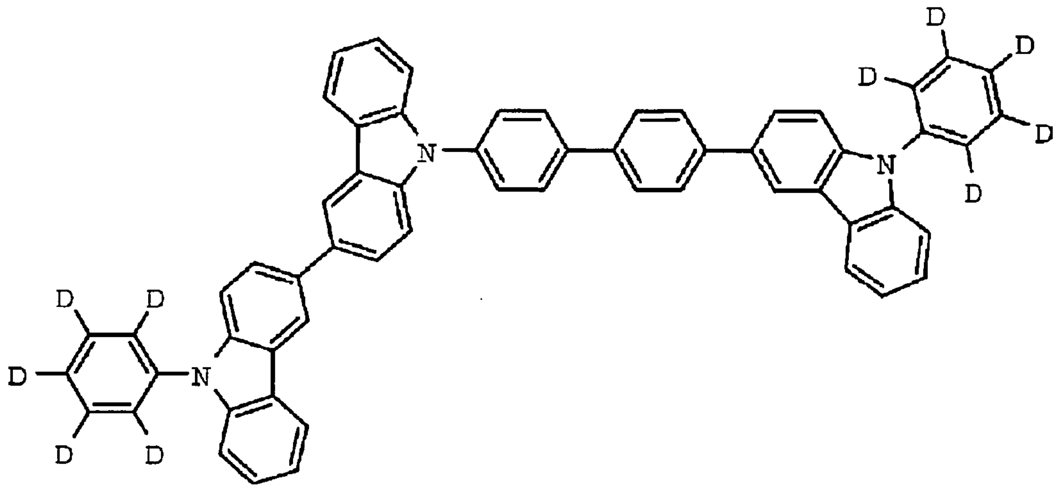

(Compound 50)

[Chemical Formula 61]

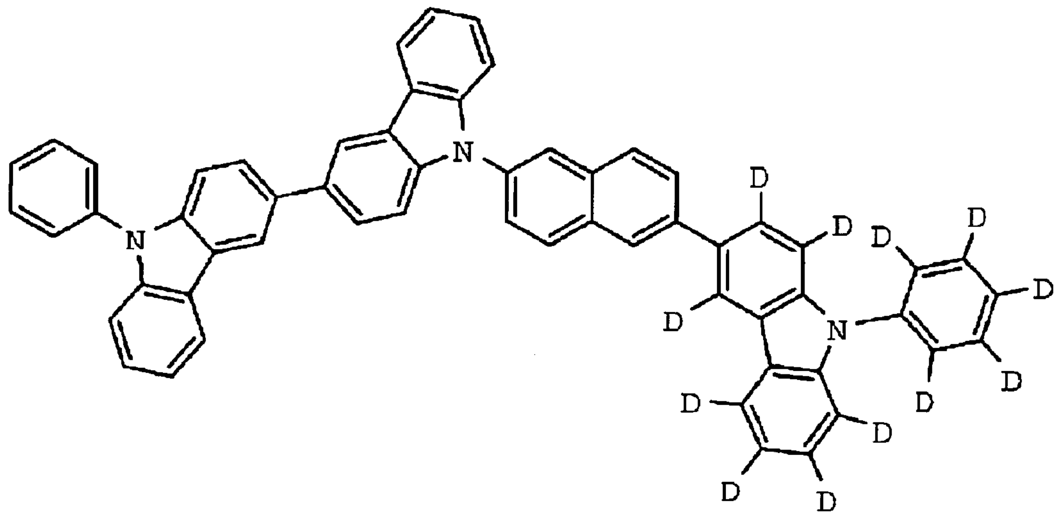

(Compound 51)

[Chemical Formula 62]

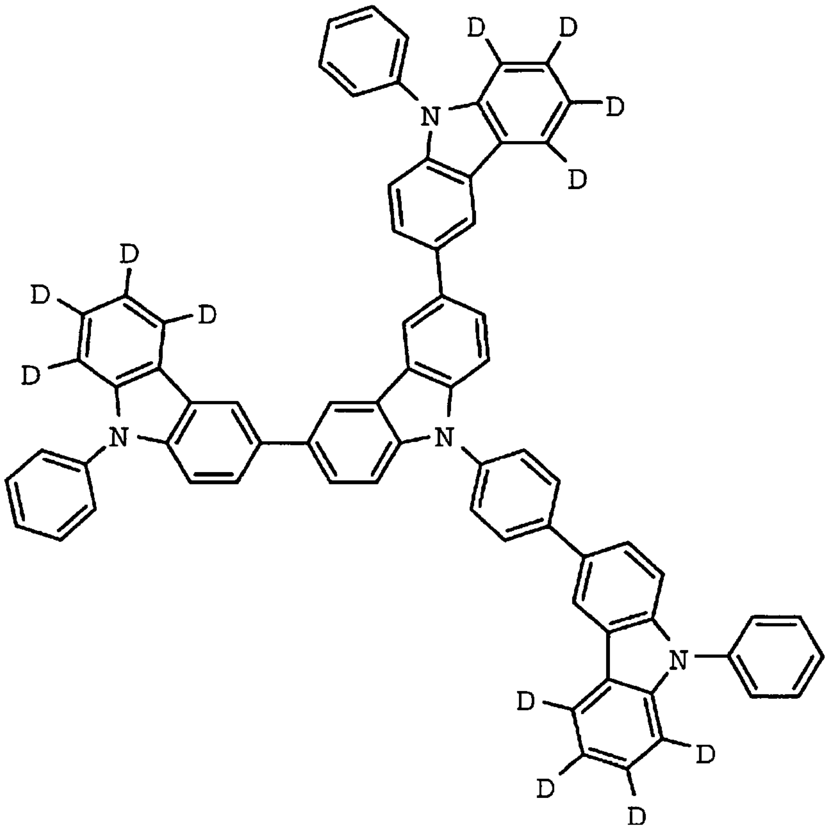

(Compound 52)

[Chemical Formula 63]

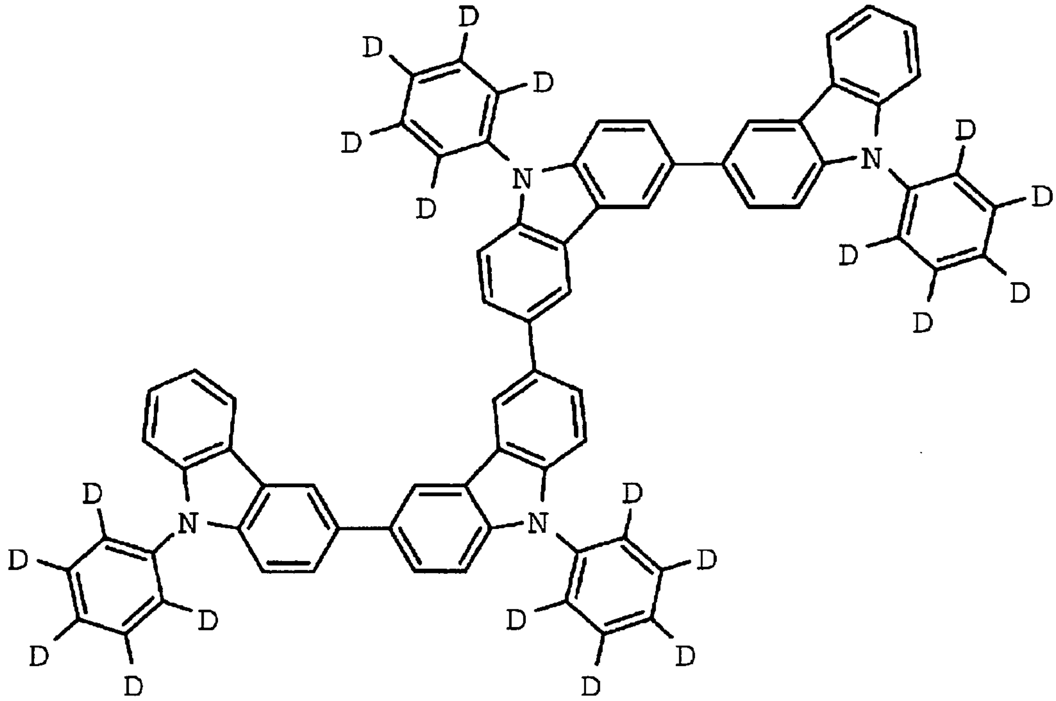

(Compound 53)

[Chemical Formula 64]

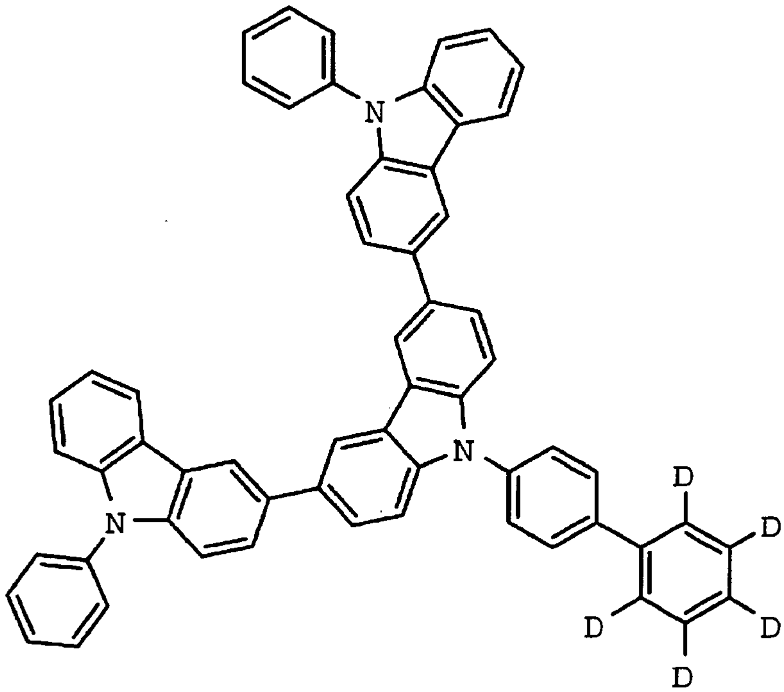

(Compound 54)

These compounds were purified by methods such as column chromatography, adsorption using, for example,

a silica gel, activated carbon, or activated clay, and recrystallization or crystallization using a solvent. The compounds were identified by NMR analysis. Glass transition point (Tg) and work function were taken for the measurement of physical properties. Glass transition point (Tg) can be used as an index of stability in the thin-film state, and the work function as an index of hole transportability.

The glass transition point (Tg) was measured using a powder, using a high-sensitive differential scanning calorimeter DSC3100S produced by Bruker AXS.

For the measurement of work function, a 100 nm-thick thin film was fabricated on an ITO substrate, and an atmosphere photoelectron spectrometer AC-3 produced by Riken Keiki Co., Ltd. was used.

The $T_1$ values of these compounds can be calculated from the measured phosphorescence spectrum. The phosphorescence spectrum can be measured using a commercially available spectrophotometer. Typically, the phosphorescence spectrum is measured by shining excitation light under low temperature on the compound dissolved in a solvent (see, for example, Non-Patent Document 9), or by shining excitation light under low temperature on the compound formed into a thin film by being vapor deposited on a silicon substrate (see, for example, Patent Document 8). $T_1$ can be calculated by conversion into a light energy value according to the equation below from the wavelength of the first peak on the shorter wavelength side of the phosphorescence spectrum, or from the wavelength at the rise of the spectrum on the shorter wavelength side. $T_1$ is used as an index of triplet exciton confinement by the phosphorescent material.

[Equation 1]

$$E(eV) = hc/\lambda$$

In the equation, E represents the light energy value, h the Planck's constant ($6.63 \times 10^{-34}$ Js), c the speed of light ($3.00 \times 10^{8}$ m/s), and $\lambda$ the wavelength (nm) at the rise of the phosphorescence spectrum on the shorter wavelength side. 1 eV = $1.60 \times 10^{-19}$ J.

The organic EL device of the present invention may have a structure including an anode, a hole injection layer, a hole transport layer, an electron blocking layer, a light emitting layer, a hole blocking layer, an electron transport layer, and a cathode successively formed on a substrate, optionally with an electron injection layer between the electron transport layer and the cathode. Some of the organic layers in this multilayer structure may be omitted.

Each of the light emitting layer, the hole transport layer, and the electron transport layer may have a laminate structure of two or more layers.

Electrode materials with a large work function, such as ITO and gold, are used as the anode of the organic EL device of the present invention. The hole injection layer of the organic EL device of the present invention may be made of a material, the examples of which include porphyrin compounds as represented by copper phthalocyanine, starburst-type triphenylamine derivatives, triphenylamine trimers and tetramers such as an arylamine compound of a structure in which three or more triphenylamine structures are joined to each other within the molecule via a single bond or a divalent group that does not contain a heteroatom, accepting heterocyclic compounds such as hexacyano azatriphenylene, and coating-type polymer materials, in addition to the compounds of general formula (1) having a carbazole ring structure of the present invention. These materials may be formed into a thin film by using a vapor deposition method, or other known methods such as spin coating and an inkjet method.

Examples of the material used for the hole transport layer of the organic EL device of the present invention include benzidine derivatives (such as TPD, $\alpha$-NPD, and N,N,N',N'-tetrabiphenylylbenzidine), TAPC, and various triphenylamine trimers and tetramers, in addition to the compounds of general formula (1) having a carbazole ring structure of the present invention. These may be individually deposited for film forming, or may be used as a single layer deposited as a mixture with other materials, or as a laminate of individually deposited layers, a laminate of layers deposited as a mixture, or a laminate of layers deposited by being mixed with an individually deposited layer. Examples of the material used for the hole injection/transport layer include coating-type polymer materials such as poly(3,4-ethylenedioxythiophene) (hereinafter, simply "PEDOT")/poly(styrene sulfonate) (hereinafter, simply "PSS"). These materials may be formed into a thin-film by using a vapor deposition method, or other known methods such as spin coating and an inkjet method.

Further, the hole injection layer or the hole transport layer may be one obtained by the P-doping of material such as trisbromophenylamine hexachloroantimony in the material commonly used for these layers. Further, for example, polymer compounds having a TPD structure as a part of the compound structure also may be used.

Examples of the material used for the electron blocking layer of the organic EL device of the present invention include compounds having an electron blocking effect, including, for example, carbazole derivatives such as 4,4',4''-tri(N-carbazolyl)triphenylamine (hereinafter, simply "TCTA"), 9,9-bis[4-(carbazol-9-yl)phenyl]fluorene, 1,3-bis(carbazol-9-yl)benzene (hereinafter, simply "mCP"), and 2,2-bis(4-carbazol-9-ylphenyl)adamantane (hereinafter, simply "Ad-Cz");

and compounds having a triphenylsilyl group and a triarylamine structure, as represented by 9-[4-(carbazol-9-yl)phenyl]-9-[4-(triphenylsilyl)phenyl]-9H-fluorene, in addition to the compounds of general formula (1) having a carbazole ring structure of the present invention. These may be individually deposited for film forming, or may be used as a single layer deposited as a mixture with other materials, or as a laminate of individually deposited layers, a laminate of layers deposited as a mixture, or a laminate of layers deposited by being mixed with an individually deposited layer. These materials may be formed into a thin-film by using a vapor deposition method, or other known methods such as spin coating and an inkjet method.

Examples of the material used for the light emitting layer of the organic EL device of the present invention include various metal complexes, anthracene derivatives, bis(styryl)benzene derivatives, pyrene derivatives, oxazole derivatives, and polyparaphenylene vinylene derivatives, in addition to quinolinol derivative metal complexes such as $Alq_3$. Further, the light emitting layer may be configured from a host material and a dopant material. Examples of the host material include thiazole derivatives, benzimidazole derivatives, and polydialkyl fluorene derivatives, in addition to the foregoing light-emitting materials, and the compounds of general formula (1) having a carbazole ring structure of the present invention. Examples of the dopant material include quinacridone, coumarin, rubrene, perylene, derivatives thereof, benzopyran derivatives, rhodamine derivatives, and aminostyryl derivatives. These may be individually deposited for film forming, or may be used as a single layer deposited as a mixture with other materials, or as a laminate of individually deposited layers, a laminate of layers deposited as a mixture, or a laminate of layers deposited by being mixed with an individually deposited layer.

Further, the light-emitting material may be phosphorescent light-emitting material. Phosphorescent materials as metal complexes of metals such as iridium and platinum may be used as the phosphorescent light-emitting material. Examples of the phosphorescent materials include green phosphorescent materials such as $Ir(ppy)_3$, blue phosphorescent materials such as FIrpic and $FIr_6$, and red phosphorescent materials such as $Btp_2Ir(acac)$. Here, the compounds of general formula (1) having a carbazole ring structure of the present invention may be used as the hole injecting and transporting host material, in addition to carbazole derivatives such as 4,4'-di (N-carbazolyl)biphenyl (hereinafter, simply "CBP"), TCTA, and mCP. Compounds such as p-bis(triphenylsilyl)benzene (hereinafter, simply "UGH2"), and 2,2', 2''-(1,3,5-phenylene)-tris(1-phenyl-1H-benzimidazole) (hereinafter, simply "TPBI") represented by the following formula may be used as the electron transporting host material.

[Chemical Formula 65]

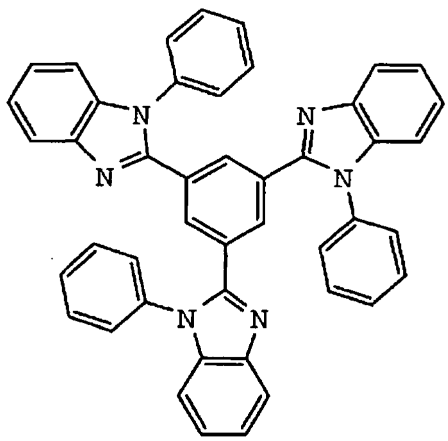

In order to avoid concentration quenching, the doping of the host material with the phosphorescent light-emitting material should preferably be made by coevaporation in a range of 1 to 30 weight percent with respect to the whole light emitting layer.

A device including a light emitting layer fabricated with the compound of general formula (1) having a carbazole ring structure used for the organic EL device of the present invention may be produced as a laminate with an adjacently laminated light emitting layer fabricated by using a compound of a different work function as the host material (see, for example, Non-Patent Documents 10 and 11).

These materials may be formed into a thin-film by using a vapor deposition method, or other known methods such as spin coating and an inkjet method.

The hole blocking layer of the organic EL device of the present invention may be formed by using hole blocking compounds such as various rare earth complexes, oxazole derivatives, triazole derivatives, and triazine derivatives, in

addition to the metal complexes of phenanthroline derivatives such as bathocuproin (hereinafter, simply "BCP"), and the metal complexes of quinolinol derivatives such as aluminum(III) bis(2-methyl-8-quinolinate)-4-phenylphenolate (hereinafter, simply "BAlq"). These materials may also serve as the material of the electron transport layer. These may be individually deposited for film forming, or may be used as a single layer deposited as a mixture with other materials, or as a laminate of individually deposited layers, a laminate of layers deposited as a mixture, or a laminate of layers deposited by being mixed with an individually deposited layer. These materials may be formed into a thin-film by using a vapor deposition method, or other known methods such as spin coating and an inkjet method.

Examples of the material used for the electron transport layer of the organic EL device of the present invention include various metal complexes, triazole derivatives, triazine derivatives, oxadiazole derivatives, thiadiazole derivatives, carbodiimide derivatives, quinoxaline derivatives, phenanthroline derivatives, and silole derivatives, in addition to quinolinol derivative metal complexes such as $Alq_3$ and BAlq. These may be individually deposited for film forming, or may be used as a single layer deposited as a mixture with other materials, or as a laminate of individually deposited layers, a laminate of layers deposited as a mixture, or a laminate of layers deposited by being mixed with an individually deposited layer. These materials may be formed into a thin-film by using a vapor deposition method, or other known methods such as spin coating and an inkjet method.

Examples of the material used for the electron injection layer of the organic EL device of the present invention include alkali metal salts (such as lithium fluoride, and cesium fluoride), alkaline earth metal salts (such as magnesium fluoride), and metal oxides (such as aluminum oxide). However, the electron injection layer may be omitted upon preferably selecting the electron transport layer and the cathode.

The electron injection layer or the electron transport layer may be one obtained by the N-doping of metals such as cesium in the materials commonly used for these layers.

The cathode of the organic EL device of the present invention may be made of an electrode material having a low work function (such as aluminum), or an alloy of an electrode material having an even lower work function (such as a magnesium-silver alloy, a magnesium-indium alloy, or an aluminum-magnesium alloy).

The thickness of each layer in the organic EL device of the present invention is not particularly limited, and is typically from 0.1 nm to 1 μm, preferably 0.3 nm to 500 nm, because defects such as pinholes are likely to occur when the layers are thin, and because applied voltage tends to increase with thick layers.

The following describes an embodiment of the present invention in more detail based on Examples. The present invention, however, is not restricted to the following Examples.

Example 1

Synthesis of 3,6-bis[9'-(phenyl-$d_5$)-9'H-carbazol-3-yl]-9-(phenyl-$d_5$)-9H-carbazole (compound 11)

3,6-Bis(9'H-carbazol-3-yl)-9H-carbazole (2.4 g) synthesized by the coupling reaction of 3-bromocarbazole and 3-(4,4,5,5-tetramethyl-1,3,2-dioxaborolan-2-yl)-9H-carbazole, bromobenzene-$d_5$ (2.3 g), palladium acetate (77 mg), sodium tert-butoxide (1.65 g), and toluene (74 ml) were added to an argon-substituted reaction vessel, and aerated with argon gas for 30 min under ultrasonic irradiation. The mixture was heated after adding 0.3 ml of tri-tert-butyl phosphine, and stirred at 90°C for 12 hours. The mixture was then cooled to 50°C, and 5 ml of methanol was added. The insoluble matter was removed by filtration, and the filtrate was concentrated under reduced pressure. The product was dissolved by addition of toluene (70 ml), and purified by adsorption using a silica gel (7 g). After concentrating the product, methanol (50 ml) was added to precipitate the crystals. The crystals were purified three times by recrystallization using toluene/n-hexane. The product was further purified by being dispersed and washed under heat using ethyl acetate (20 ml) to obtain a crude product. The crude product was dissolved by adding toluene (50 ml), and purified by adsorption using an activated clay (1 g). The product was then crystallized with methanol to obtain a white powder of 3,6-bis[9'-(phenyl-$d_5$)-9'H-carbazol-3-yl]-9-(phenyl-$d_5$)-9H-carbazole (1.96 g; yield 47%).

The structure of the resulting brownish white powder was identified by NMR. The $^1$H-NMR measurement result is presented in FIG. 1.

$^1$H-NMR (THF-$d_8$) detected 20 hydrogen signals, as follows. δ (ppm) = 8.70(2H), 8.60(2H), 8.28(2H), 7.86-7.83(4H), 7.54-7.50(4H), 7.43-7.37(4H), 7.27(2H).

Example 2

Synthesis of 3,6-bis(9'-phenyl-9'H-carbazol-3-yl)-9-(phenyl-$d_5$)-9H-carbazole (compound 12)

3,6-Dibromo-9-(phenyl-$d_5$)-9H-carbazole (26.1 g) synthesized by bromination after the coupling reaction of 9H-carbazole and bromobenzene-$d_5$, 9-phenyl-3-(4,4,5,5-tetramethyl-1,3,2-dioxaborolan-2-yl)-9H-carbazole (48.7 g), toluene (326 ml), ethanol (82 ml), and a 2M potassium carbonate aqueous solution (95 ml) were added to a nitrogen-substituted reaction vessel, and aerated with nitrogen gas for 30 min under ultrasonic irradiation. The mixture was heated after adding tetrakis(triphenylphosphine)palladium (2.23 g), and stirred at 72°C for 6.5 hours. The mixture was then allowed to cool to room temperature. After adding methanol (650 ml), the precipitated crude product was collected by filtration. The crude product was dissolved by addition of toluene (1,130 ml), and purified by adsorption using a diamine silica gel (18.5 g), and then by adsorption using a silica gel (18.5 g). The product was concentrated under reduced pressure, and purified three times by recrystallization using toluene/n-hexane. The product was then purified by being dispersed and washed under heat using methanol to obtain a white powder of 3,6-bis(9'-phenyl-9'H-carbazol-3-yl)-9-(phenyl-$d_5$)-9H-carbazole (32.3 g; yield 69%).

The structure of the resulting white powder was identified by NMR. The $^1$H-NMR measurement result is presented in FIG. 2.

$^1$H-NMR (THF-$d_8$) detected 30 hydrogen signals, as follows. $\delta$ (ppm) = 8.70(2H), 8.60(2H), 8.28(2H), 7.86-7.83(4H), 7.66-7.65(8H), 7.54-7.49(6H), 7.42-7.36(4H), 7.27(2H).

Example 3

Synthesis of 3,6-bis(9'-phenyl-9'H-carbazol-3-yl)-9-[4-(phenyl-$d_5$)phenyl]-9H-carbazole (compound 54)

3,6-Dibromo-9-[4-(phenyl-$d_5$)phenyl]-9H-carbazole (4.50 g) synthesized by bromination after the coupling reaction of 9H-carbazole and 1-bromo-4-(phenyl-$d_5$)benzene, 9-phenyl-3-(4,4,5,5-tetramethyl-1,3,2-dioxaborolan-2-yl)-9H-carbazole (7.06 g), toluene (67.5 ml), ethanol (17 ml), and a 2M potassium carbonate aqueous solution (14 ml) were added to a nitrogen-substituted reaction vessel, and aerated with nitrogen gas for 30 min under ultrasonic irradiation. The mixture was heated after adding tetrakis(triphenylphosphine)palladium (324.4 mg), and stirred at 72°C for 8.5 hours. The mixture was then allowed to cool to room temperature. After adding methanol (130 ml), the precipitated crude product was collected by filtration. The crude product was dissolved by adding toluene (225 ml), and purified by adsorption using a diamine silica gel (3.5 g), and then by adsorption using a silica gel (7.5 g). The product was concentrated under reduced pressure, and purified twice by recrystallization using toluene/methanol, and twice by recrystallization using 1,2-dichlorobenzene. The product was then purified by being dispersed and washed under heat using methanol to obtain a white powder of 3,6-bis(9'-phenyl-9'H-carbazol-3-yl)-9-[4-(phenyl-$d_5$)phenyl]-9H-carbazole (4.22 g; yield 56%).

The structure of the resulting white powder was identified by NMR. The $^1$H-NMR measurement result is presented in FIG. 3.

$^1$H-NMR (THF-$d_8$) detected 34 hydrogen signals, as follows. $\delta$ (ppm) = 8.71(2H), 8.61(2H), 8.28(2H), 7.95(2H), 7.86-7.84(4H), 7.77(2H), 7.65-7.60(8H), 7.58(2H), 7.50-7.47(4H), 7.42-7.37(4H), 7.27(2H).

Example 4

The glass transition points of the compounds used in the present invention, and the glass transition point of comparative compound 55 of the structural formula below were determined using a high-sensitive differential scanning calorimeter DSC 3100S produced by Bruker AXS.

| | Glass transition point |
|---|---|
| Compound of Example 1 of the present invention | 155.0°C |
| Compound of Example 2 of the present invention | 159.7°C |
| Compound of Example 3 of the present invention | 163.8°C |
| Comparative compound 55 | 142.5°C |

[Chemical Formula 66]

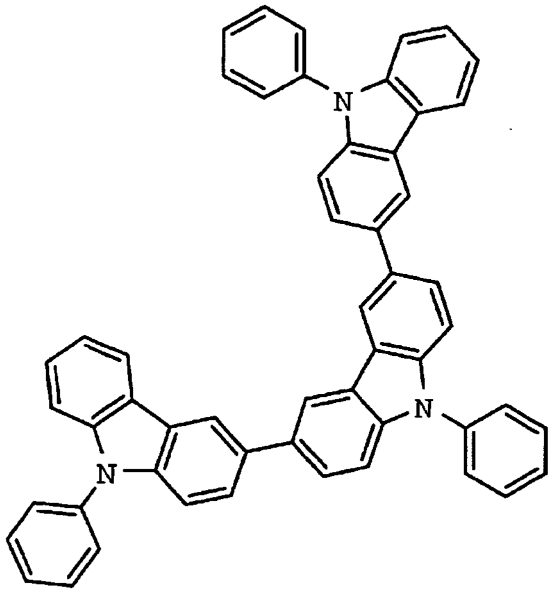

(Comparative Compound 55)

The compounds used in the present invention have glass transition points of 100°C or higher, demonstrating that the compounds used in the present invention have a stable thin-film state.
The results also demonstrate that the substitution with a deuterium atom can improve heat resistance and thin-film stability.

Example 5

A 100 nm-thick vapor-deposited film was fabricated on an ITO substrate using the compounds used in the present invention, and the comparative compound 55 of the structural formula above. The work function was measured using an atmosphere photoelectron spectrometer (Model AC-3 produced by Riken Keiki Co., Ltd.).

| | Work function |
|---|---|
| Compound of Example 1 of the present invention | 5.46 eV |
| Compound of Example 2 of the present invention | 5.45 eV |
| Compound of Example 3 of the present invention | 5.60 eV |
| Comparative Compound 55 | 5.44 eV |

As the results show, the compounds used in the present invention have desirable energy levels compared to the work function 5.4 eV of common hole transport materials such as α-NPD and TPD, and thus possess desirable hole transportability.

Example 6

A 1.0 × $10^{-5}$ mol/L 2-methyltetrahydrofuran solution was prepared for the compounds used in the present invention. The prepared solution was placed in a designated quartz tube, and aerated with pure nitrogen to remove the oxygen content. The tube was plugged with a septum rubber to prevent mixing with oxygen. After being cooled to 77 K, the solution was irradiated with excitation light to measure the phosphorescence spectrum, using a spectrofluorometer FluoroMax-4 produced by Horiba Ltd. The wavelength of the first peak on the shorter wavelength side of the phosphorescence spectrum was taken, and the wavelength value was converted to light energy to calculate $T_1$.

| | $T_1$ |
|---|---|
| Compound of Example 1 of the present invention | 2.74 eV |
| Compound of Example 2 of the present invention | 2.75 eV |

(continued)

| | $T_1$ |
|---|---|
| Compound of Example 3 of the present invention | 2.68 eV |
| CBP | 2.56 eV |
| FIrpic | 2.62 eV |
| $Ir(ppy)_3$ | 2.42 eV |
| α-NPD | 2.29 eV |

As can be seen, the compounds used in the present invention have higher $T_1$ values than the commonly used blue phosphorescent material FIrpic, green phosphorescent material $Ir(ppy)_3$, the commonly used host material CBP, and the commonly used hole transport material α-NPD, and thus have sufficient capability for the confinement of the triplet excitons excited in the light emitting layer.

Example 7

The organic EL device, as illustrated in FIG. 4, was fabricated from a hole transport layer 3, a light emitting layer 4, a hole blocking layer 5, an electron transport layer 6, an electron injection layer 7, and a cathode (aluminum electrode) 8 successively formed by vapor deposition on a glass substrate 1 that had been provided beforehand with an ITO electrode as a transparent anode 2.

Specifically, the glass substrate 1 having ITO (thickness 150 nm) formed thereon was washed with an organic solvent, and subjected to an oxygen plasma treatment to wash the surface. The glass substrate with the ITO electrode was then installed in a vacuum vapor deposition apparatus, and the pressure was reduced to 0.001 Pa or less. This was followed by formation of the hole transport layer 3 by forming the compound of Example 1 of the present invention (Compound 11) over the transparent anode 2 in a thickness of 50 nm. Thereafter, the light emitting layer 4 was formed on the hole transport layer 3 by forming TPBI and $Ir(ppy)_3$ in a thickness of 20 nm using dual vapor deposition at a deposition rate ratio of TPBI:$Ir(ppy)_3$ = 92:8. The hole blocking layer 5 was then formed on the light emitting layer 4 by forming BCP in a thickness of 10 nm. Then, the electron transport layer 6 was formed on the hole blocking layer 5 by forming $Alq_3$ in a thickness of 30 nm. The electron injection layer 7 was then formed on the electron transport layer 6 by forming lithium fluoride in a thickness of 0.5 nm. Finally, the cathode 8 was formed by vapor depositing aluminum in a thickness of 150 nm. The characteristics of the organic EL device thus fabricated were measured in an atmosphere at ordinary temperature. Table 1 summarizes the results of the emission characteristics measurements performed by applying a DC voltage to the organic EL device.

Example 8

An organic EL device was fabricated under the same conditions used in Example 7, except that the compound of Example 2 (compound 12) of the present invention was formed in a thickness of 50 nm as the material of the hole transport layer 3, instead of using the compound of Example 1 (compound 11) of the present invention. The characteristics of the organic EL device thus fabricated were measured in an atmosphere at ordinary temperature. Table 1 summarizes the results of the emission characteristics measurements performed by applying a DC voltage to the organic EL device.

Comparative Example 1

For comparison, an organic EL device was fabricated under the same conditions used in Example 7, except that the comparative compound 55 of the structural formula above was formed in a thickness of 50 nm as the material of the hole transport layer 3, instead of using the compound of Example 1 (compound 11) of the present invention. The characteristics of the organic EL device thus fabricated were measured in an atmosphere at ordinary temperature. Table 1 summarizes the results of the emission characteristics measurements performed by applying a DC voltage to the organic EL device.

Comparative Example 2

For comparison, an organic EL device was fabricated under the same conditions used in Example 7, except that α-NPD was formed in a thickness of 50 nm as the material of the hole transport layer 3, instead of using the compound of Example 1 (compound 11) of the present invention. The characteristics of the organic EL device thus fabricated were measured in an atmosphere at ordinary temperature. Table 1 summarizes the results of the emission characteristics

measurements performed by applying a DC voltage to the organic EL device.

Table 1

| | Hole transport layer material | Voltage [V] (@10mA/cm$^2$) | Luminance [cd/m$^2$] (@10mA/cm$^2$) | Current efficiency [cd/A] (@10mA/cm$^2$) | Power efficiency [1m/W] (@10mA/cm$^2$) |
|---|---|---|---|---|---|
| Ex. 7 | Compound 11 | 5.55 | 3184 | 31.87 | 18.04 |
| Ex. 8 | Compound 12 | 5.44 | 3095 | 30.97 | 17.89 |
| Com. Ex. 1 | Comparative compound 55 | 5.41 | 3039 | 30.41 | 17.65 |
| Com. Ex. 2 | α-NPD | 6.54 | 1931 | 19.33 | 9.29 |

As can be seen in Table 1, the driving voltage upon passing a current with a current density of 10 mA/cm$^2$ was 5.44 to 5.55 V for the compounds of Examples 1 and 2 of the present invention (compounds 11 and 12) used as the material of the hole transport layer, about the same as the driving voltage 5.41 V for the comparative compound 55, and much lower than 6.54 V of when α-NPD was used. Further, the emission luminance, the current efficiency, and the power efficiency all greatly improved when the compounds of Examples 1 and 2 of the present invention (Compounds 11 and 12) were used than when α-NPD was used. The emission luminance, the current efficiency, and the power efficiency also improved compared with the comparative compound 55.

The results of turn on voltage measurements using the foregoing organic EL devices are presented below.

| Organic EL device | Hole transport layer material | Turn on voltage [V] |
|---|---|---|
| Example 7 | Compound 11 | 2.7 |
| Example 8 | Compound 12 | 2.7 |
| Comparative Example 1 | Comparative compound 55 | 2.7 |
| Comparative Example 2 | α-NPD | 2.9 |

It can be seen that Examples 7 and 8 maintained the turn on voltages about the same as that of Comparative Example 1 in which comparative compound 55 was used, and that the turn on voltage was lower in Examples 7 and 8 than in Comparative Example 2 in which α-NPD was used.

As these results demonstrate, the organic EL devices in which the compound of general formula (1) having a carbazole ring structure used in the present invention is used as the material of the hole transport layer can have greatly improved emission luminance, luminous efficiency, and power efficiency, compared to the organic EL devices in which the common α-NPD is used as the material of the hole transport layer.

It was also found that the emission luminance, the luminous efficiency, and the power efficiency can be improved over the organic EL device in which the comparative compound 55 unsubstituted with a deuterium atom is used as the material of the hole transport layer.

Industrial Applicability

The organic EL device produced by using the compound of the general formula (1) having a carbazole ring structure can have high emission luminance, high luminous efficiency, and high power efficiency, and can have a low actual driving voltage to improve durability. There are potential applications for, for example, home electronic appliances and illuminations.

Description of Reference Numerals and Signs

| | |
|---|---|
| 1 | Glass substrate |
| 2 | Transparent anode |
| 3 | Hole transport layer |
| 4 | Light emitting layer |

(continued)

| | |
|---|---|
| 5 | Hole blocking layer |
| 6 | Electron transport layer |
| 7 | Electron injection layer |
| 8 | Cathode |

## Claims

1. A compound of the following general formula (1) having a carbazole ring structure,

[Chemical Formula 1]

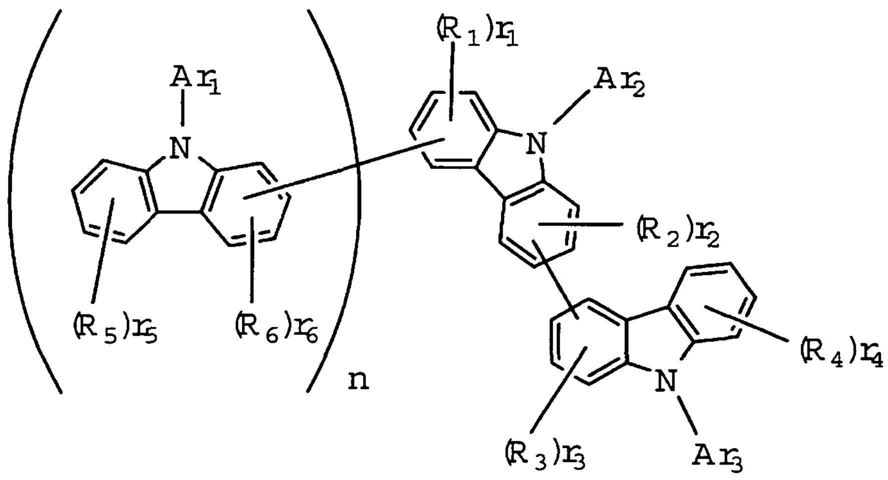

(1)

wherein $R_1$, $R_2$, $R_3$, $R_4$, $R_5$, and $R_6$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, $r_1$, $r_4$, and $r_5$ may be the same or different, and represent 0 or an integer of 1 to 4, $r_2$, $r_3$, and $r_6$ may be the same or different, and represent 0 or an integer of 1 to 3, n represents 0 or an integer of 1, $Ar_1$, $Ar_2$, and $Ar_3$ may be the same or different, and represent a substituted or unsubstituted aromatic hydrocarbon group, a substituted or unsubstituted aromatic heterocyclic group, or a substituted or unsubstituted condensed polycyclic aromatic group, and wherein at least one of $R_1$ to $R_6$, or at least one of the substituents of $Ar_1$ to $Ar_3$ is a deuterium atom, or a substituent that contains a deuterium atom.

2. The compound having a carbazole ring structure according to claim 1, wherein $Ar_2$ in the general formula (1) is a monovalent group represented by the following general formula (2) or (3),

[Chemical Formula 2]

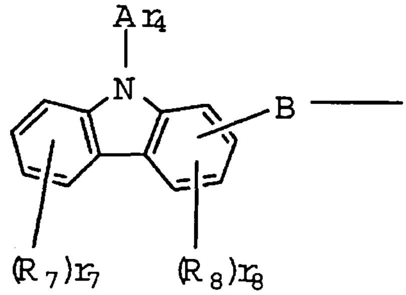

(2)

wherein $R_7$ and $R_8$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, $r_7$ represents 0 or an integer of 1 to 4, $r_8$ represents 0 or an integer of 1 to 3, B represents a divalent group of a substituted or unsubstituted aromatic hydrocarbon group, a divalent group of a substituted or unsubstituted aromatic heterocyclic ring, or a divalent group of a substituted or unsubstituted condensed polycyclic aromatic group, $Ar_4$ represents a substituted or unsubstituted aromatic hydrocarbon group, a substituted or unsubstituted aromatic heterocyclic group, or a substituted or unsubstituted condensed polycyclic aromatic group, and wherein at least one of $R_7$ and $R_8$, or at least one of the substituents of $Ar_4$ or B is a deuterium atom, or a substituent that contains a deuterium atom,

[Chemical Formula 3]

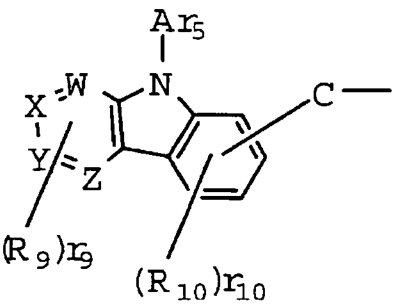

(3)

wherein $R_9$ and $R_{10}$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, $r_9$ and $r_{10}$ may be the same or different, and represent 0 or an integer of 1 to 3, C represents a divalent group of a substituted or unsubstituted aromatic hydrocarbon group, a divalent group of a substituted or unsubstituted aromatic heterocyclic ring, or a divalent group of a substituted or unsubstituted condensed polycyclic aromatic group, $Ar_5$ represents a substituted or unsubstituted aromatic hydrocarbon group, a substituted or unsubstituted aromatic heterocyclic group, or a substituted or unsubstituted condensed polycyclic aromatic group, W, X, Y, and Z represent a carbon atom or a nitrogen atom, where only one of W, X, Y, and Z is a nitrogen atom, and, in this case, the nitrogen atom does not have the substituent $R_9$, and wherein at least one of $R_9$ and $R_{10}$, or at least one of the substituents of $Ar_5$ or C is a deuterium

atom, or a substituent that contains a deuterium atom.

**3.** The compound having a carbazole ring structure according to claim 1 or 2, wherein n in the general formula (1) is 0.

**4.** The compound having a carbazole ring structure according to claim 1 or 2, wherein n in the general formula (1) is 1.

**5.** The compound having a carbazole ring structure according to claim 4, wherein the compound is represented by the following general formula (1'),

[Chemical Formula 4]

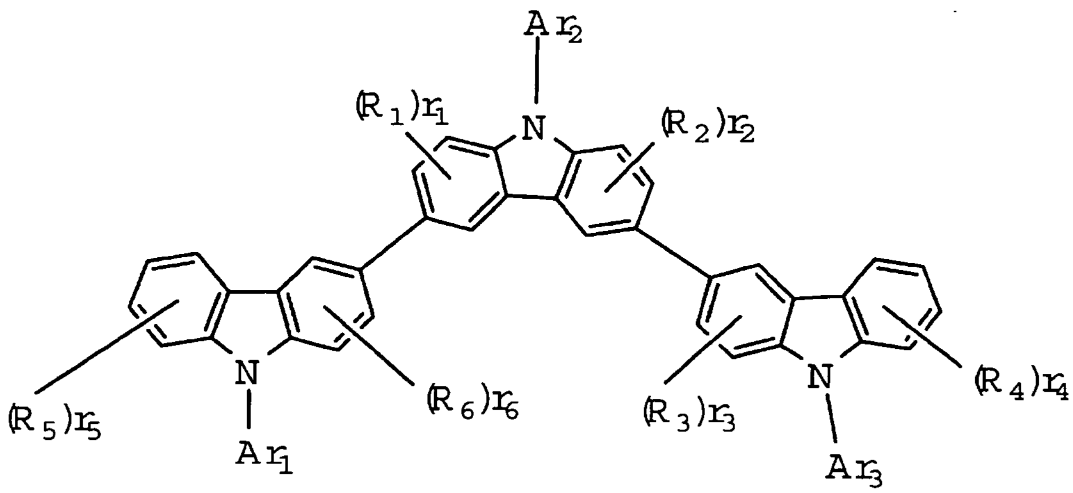

(1')

wherein $R_1$, $R_2$, $R_3$, $R_4$, $R_5$, and $R_6$ may be the same or different, and represent a deuterium atom, a fluorine atom, a chlorine atom, cyano, trifluoromethyl, nitro, linear or branched alkyl of 1 to 6 carbon atoms that may have a substituent, cycloalkyl of 5 to 10 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, $r_4$ and $r_5$ may be the same or different, and represent 0 or an integer of 1 to 4, $r_1$, $r_2$, $r_3$, and $r_6$ may be the same or different, and represent 0 or an integer of 1 to 3, $Ar_1$, $Ar_2$, and $Ar_3$ may be the same or different, and represent a substituted or unsubstituted aromatic hydrocarbon group, a substituted or unsubstituted aromatic heterocyclic group, or a substituted or unsubstituted condensed polycyclic aromatic group, and wherein at least one of $R_1$ to $R_6$, or at least one of the substituents of $Ar_1$ to $Ar_3$ is a deuterium atom, or a substituent that contains a deuterium atom.

**6.** An organic electroluminescent device that comprises a pair of electrodes, and one or more organic layers sandwiched between the pair of electrodes,
wherein the compound having a carbazole ring structure of any one of claims 1 to 5 is used as a constituent material of at least one organic layer.

**7.** The organic electroluminescent device according to claim 6, wherein the organic layer is a hole transport layer.

**8.** The organic electroluminescent device according to claim 6, wherein the organic layer is a hole injection layer.

**9.** The organic electroluminescent device according to claim 6, wherein the organic layer is an electron blocking layer.

**10.** The organic electroluminescent device according to any one of claims 6 to 9, further comprising a light emitting layer that contains a phosphorescent light-emitting material.

**11.** The organic electroluminescent device according to claim 10, wherein the phosphorescent light-emitting material is a metal complex that contains iridium or platinum.

FIG.1

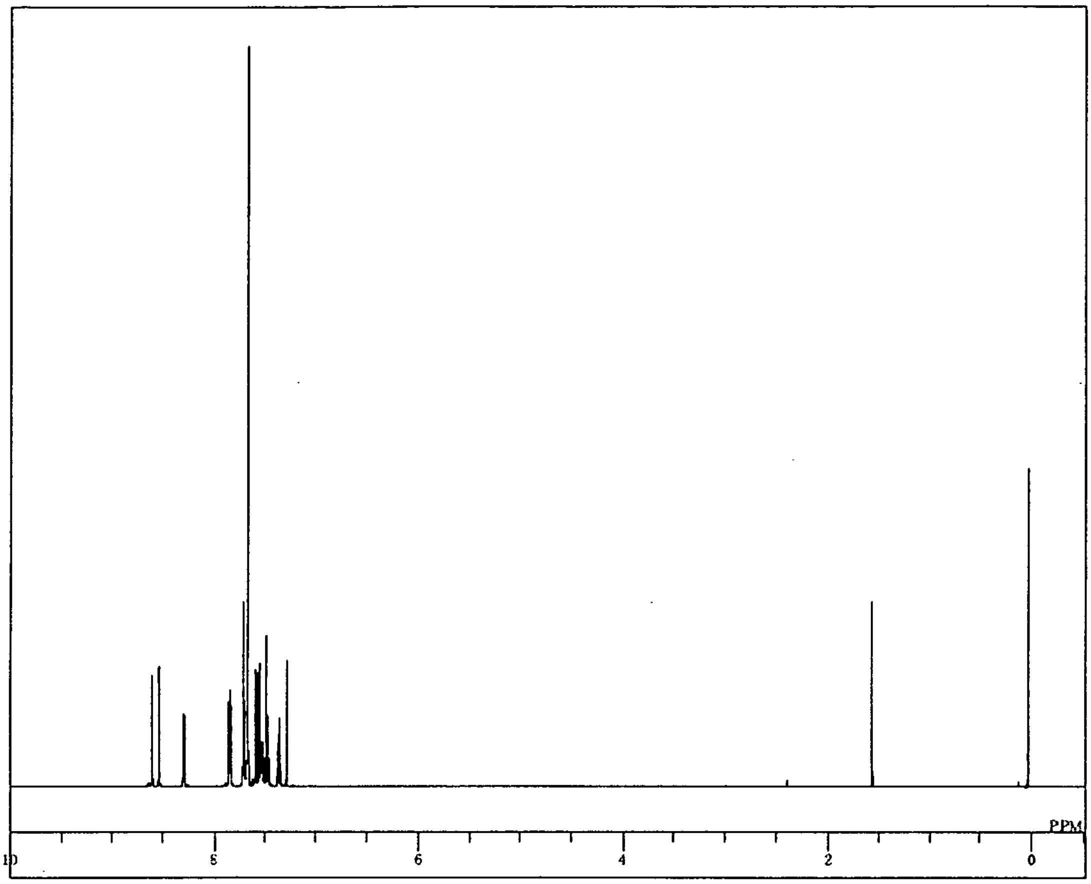

FIG.2

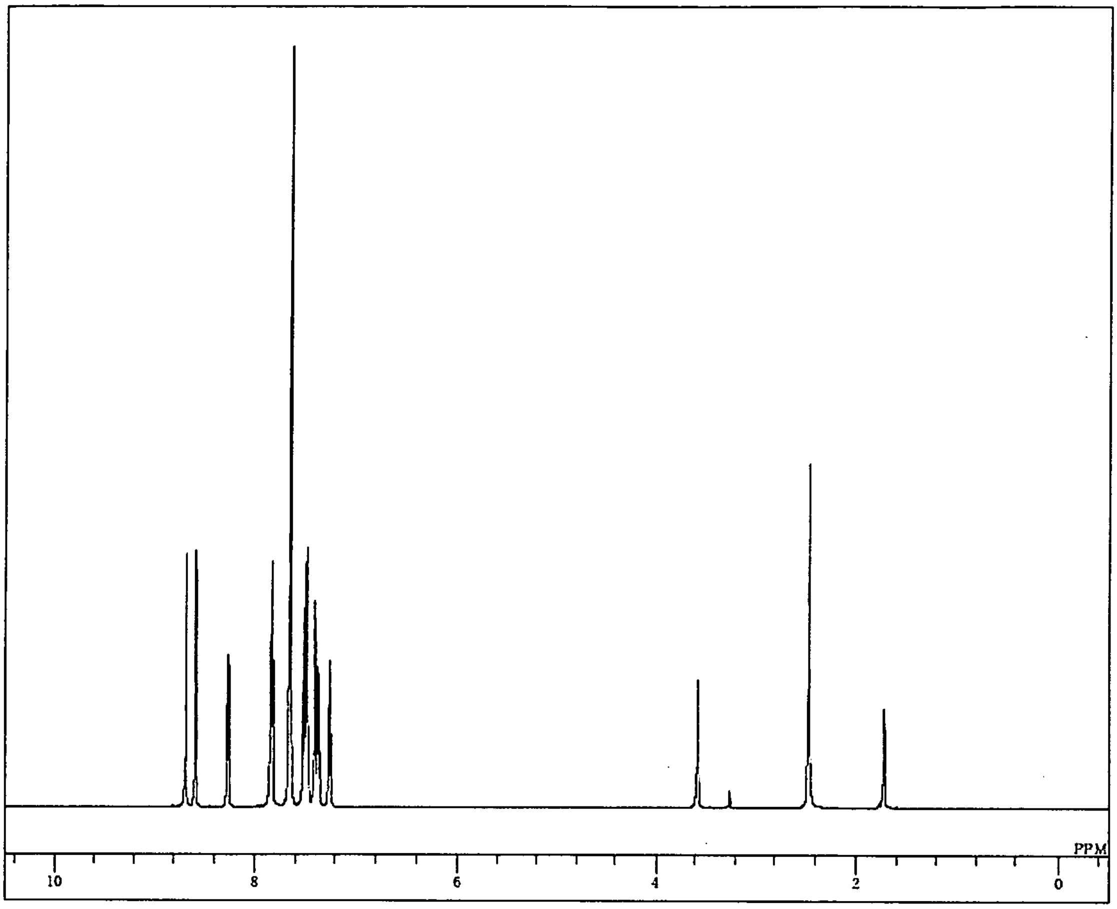

FIG.3

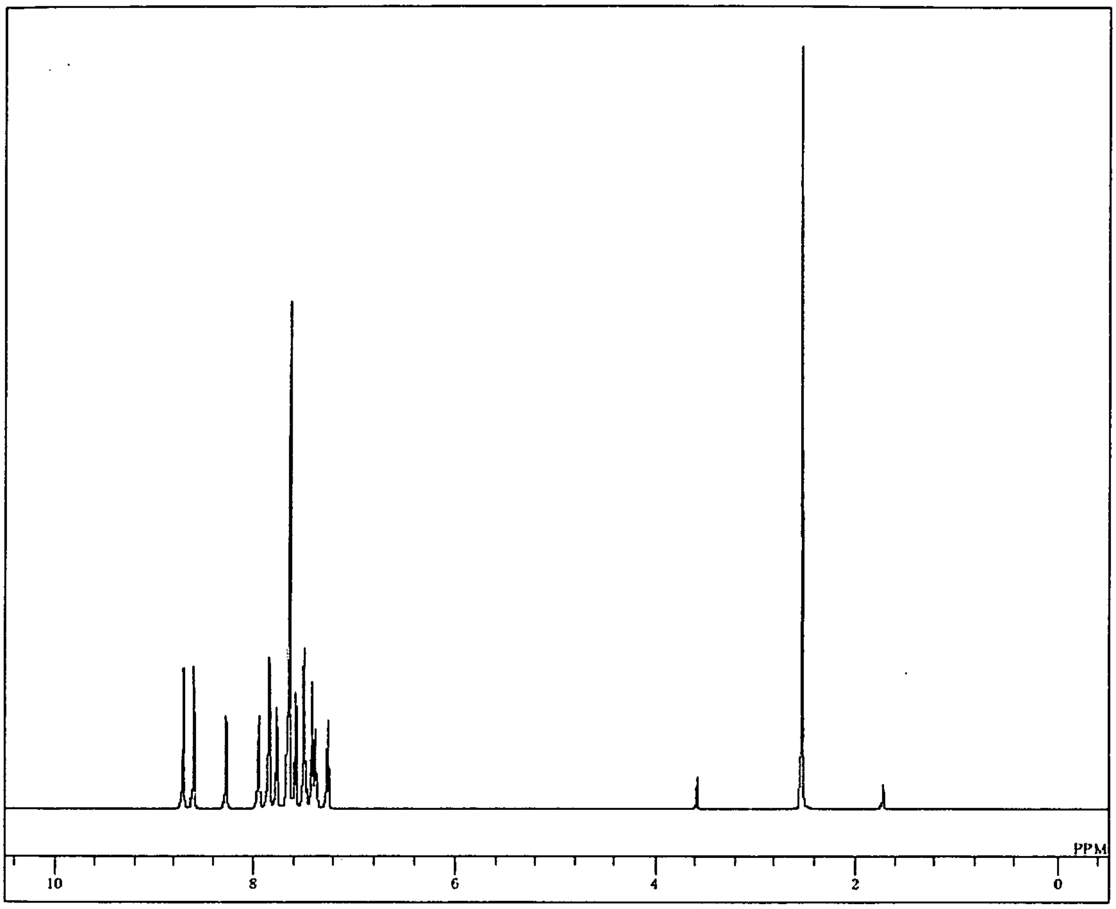
PPM
10
8
6
4
2
0

FIG.4

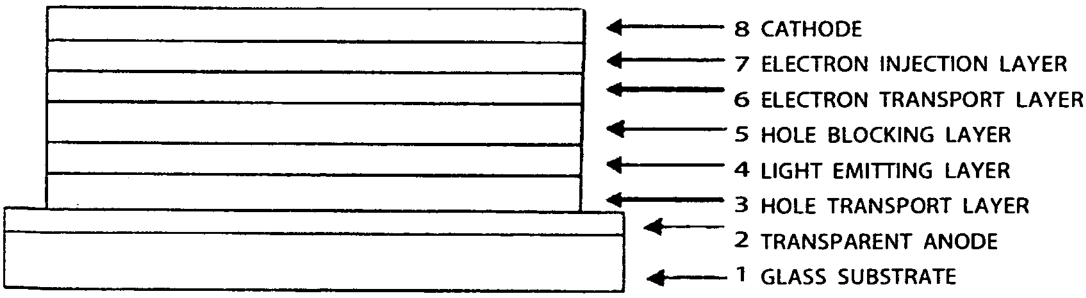
8 CATHODE
7 ELECTRON INJECTION LAYER
6 ELECTRON TRANSPORT LAYER
5 HOLE BLOCKING LAYER
4 LIGHT EMITTING LAYER
3 HOLE TRANSPORT LAYER
2 TRANSPARENT ANODE
1 GLASS SUBSTRATE

**INTERNATIONAL SEARCH REPORT**

International application No.
PCT/JP2011/003764

A. CLASSIFICATION OF SUBJECT MATTER
*C07D209/86*(2006.01)i, *C07D401/14*(2006.01)i, *C07D403/14*(2006.01)i, *C07D409/14*(2006.01)i, *H01L51/50*(2006.01)i

According to International Patent Classification (IPC) or to both national classification and IPC

B. FIELDS SEARCHED

Minimum documentation searched (classification system followed by classification symbols)
C07D209/86, C07D401/14, C07D403/14, C07D409/14, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 8-3547 A (Toray Industries, Inc.), 09 January 1996 (09.01.1996), cited in the specification of the present application as "patent document 3"; particularly, abstract; claims 1 to 5; compounds (7), (31) (Family: none) | 1-11 |
| Y | WO 2009/104488 A1 (KONICA MINOLTA HOLDINGS, INC.), 27 August 2009 (27.08.2009), particularly, abstract; claims 1 to 5; compounds (1)-2, (1)-12, (1)-13, (1)-17 (Family: none) | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 July, 2011 (26.07.11) | 09 August, 2011 (09.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/003764

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2009-231516 A (Fujifilm Corp.), 08 October 2009 (08.10.2009), cited in the specification of the present application as "patent document 7"; particularly, abstract; claims 1 to 5; pages 20 to 26 & US 2010/0084967 A1 & EP 2129739 A & CN 101646745 A & KR 10-2010-0014603 A & WO 2008/117889 A1 | 1-11 |
| Y | JP 2005-48004 A (Canon Inc.), 24 February 2005 (24.02.2005), cited in the specification of the present application as "patent document 6"; particularly, abstract; claims 1 to 4; compound 30 (Family: none) | 1-11 |
| Y | KR 10-2009-0086015 A (SFC CO., LTD.), 10 August 2009 (10.08.2009), particularly, abstract; claims 1 to 6 & KR 10-2010-0066424 A | 1-11 |
| Y | WO 2009/061145 A1 (LG CHEM LTD.), 14 May 2009 (14.05.2009), particularly, abstract; claims 1 to 12; formula 27 & JP 2011-503055 A & US 2010/0244008 A & EP 2215183 A & EP 2215182 A & CN 101896574 A & KR 10-2009-0048299 A & KR 10-2009-0051140 A & KR 10-2009-0051141 A & CN 101855315 A & WO 2009/061156 A1 & JP 2011-503056 A | 1-11 |
| P,X | WO 2011/019156 A1 (ROHM AND HAAS ELECTRONIC MATERIALS KOREA LTD.), 17 February 2011 (17.02.2011), particularly, abstract; claims 1 to 10; compound 66 (Family: none) | 1,3-11 |
| P,X | WO 2011/024451 A1 (Hodogaya Chemical Co., Ltd.), 03 March 2011 (03.03.2011), particularly, abstract; claims 1 to 17; compounds 72, 73, 84, 85 (Family: none) | 1-11 |
| P,Y | WO 2011/048822 A1 (Hodogaya Chemical Co., Ltd.), 28 April 2011 (28.04.2011), particularly, abstract; claims 1 to 10; compounds 5, 26; examples (Family: none) | 1-11 |

Form PCT/ISA/210 (continuation of second sheet) (July 2009)

**INTERNATIONAL SEARCH REPORT**

International application No.
PCT/JP2011/003764

C (Continuation). DOCUMENTS CONSIDERED TO BE RELEVANT

| Category* | Citation of document, with indication, where appropriate, of the relevant passages | Relevant to claim No. |
|---|---|---|
| P,Y | WO 2011/048821 A1 (Hodogaya Chemical Co., Ltd.), 28 April 2011 (28.04.2011), particularly, abstract; claims 1 to 9; compounds 4, 25; examples (Family: none) | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 8048656 A
- JP 3194657 B
- JP 8003547 A
- JP 2006151979 A
- WO 200862636 A
- JP 2005048004 A
- JP 2009231516 A
- JP 2007022986 A


### Non-patent literature cited in the description


- **C. W. TANG et al.** *Eastman Kodak,* 1987
- *The Japan Society of Applied Physics, 9th lecture preprints,* 2001, 55-61
- *The Japan Society of Applied Physics, 9th lecture preprints,* 2001, 23-31
- *J. Appl. Phys.,* 2004, vol. 12 (95), 7798
- *Appl. Phys. Lett.,* 2008, vol. 93, 063306
- *Helvetica Chimica Acta,* 2006, vol. 89, 1123
- *J. Org. Chem.,* 1995, vol. 60, 7508
- *Synth. Commun.,* 1981, vol. 11, 513
- Jikken Kagaku Kouza 7. Maruzen, 1992, 384-398
- Organic EL Symposium. *1st Regular presentation Preprints,* 2005, 19
- *Appl. Phys. Lett.,* 2008, vol. 93, 133312